# EUROPEAN PATENT SPECIFICATION

(11) **EP 1 206 288 B1**
(45) Date of publication and mention of the grant of the patent: **10.01.2007**
(21) Application number: 00950690.8
(22) Date of filing: 26.07.2000
(51) Int. Cl.: A61K 51/12, A61K 41/00, A61K 49/00

(54) **TARGETED FIBERLESS RADIATIVE EFFECTORS**
ZIELGERICHTETE FIBERLOSE RADIATIVE EFFEKTOREN
EFFECTEURS RADIATIFS CIBLES EXEMPTS DE FIBRES

(30) Priority: 02.08.1999 US 366314
(43) Date of publication of application: 22.05.2002
(73) Proprietor: THE REGENTS OF THE UNIVERSITY OF MICHIGAN, Ann Arbor, Mi 48109-1280 (US)
(72) Inventor: PHILBERT, Martin, A., Plymouth, MI 48170 (US); TJALKENS, Ronald, Ann Arbor, MI 48103 (US); AYLOTT, Jonathan, W., Ann Arbor, MI 48105 (US); CLARK, Heather, A., Middletown, CT 06457 (US); MONSON, Eric, E., Ann Arbor, MI 48104 (US); KOPELMAN, Raoul, Ann Arbor, MI 48104 (US)
(74) Representative: Glawe, Delfs, Moll
(86) International application number: PCT/US2000/020292
(87) International publication number: WO 2001/008660

(56) References cited:
- WO-A-00/43045
- WO-A-96/23524
- WO-A-97/46262
- WO-A-99/02651
- US-A- 5 599 923
- US-B- 6 027 726
- CLARK H A ET AL: "OPTOCHEMICAL NANOSENSORS AND SUBCELLAR APPLICATIONS IN LIVING CELLS" MIKROCHIMICA ACTA,SPRINGER VERLAG, VIENNA,AT, vol. 131, no. 1/02, 1999, pages 121-128, XP000992957 ISSN: 0026-3672

### SEQUENCE LISTING

<110> Philbert, Martin A.
   Tjalkens, Ronald
   Aylott, Jonathan W.
   Clark, Heather A.
   Monson, Eric E.
   Kopelman, Raoul
<120> Targeted Fiberless Radiative Effectors
<130> UM-03686
<140> 09/366,314
   <141> 1999-08-02
<160> 9
<170> PatentIn Ver. 2.0
<210> 1
   <211> 9
   <212> PRT
   <213> Artificial Sequence
<220>
   <223> Description of Artificial Sequence: Synthetic
<400> 1
   Cys Asp Cys Arg Gly Asp Cys Phe Cys
   1 5
<210> 2
   <211> 13
   <212> PRT
   <213> Artificial Sequence
<220>
   <223> Description of Artificial Sequence: Synthetic
<400> 2
   Cys Asn Gly Arg Cys Val Ser Gly Cys Ala Gly Arg Cys
   1 5 10
<210> 3
   <211> 3
   <212> PRT
   <213> Artificial Sequence
<220>
   <223> Description of Artificial Sequence: Synthetic <400> 3
<210> 4
   <211> 114
   <212> PRT
   <213> Homo sapiens
<400> 4
   Ser Pro Gly Pro Val Pro Pro Ser Thr Ala Leu Arg Glu Leu Ile Glu
    1               5                  10                  15
   Glu Leu Val Asn Ile Thr Gln Asn Gln Lys Ala Pro Leu Cys Asn Gly
               20                  25                  30
   Ser Met Val Trp Ser Ile Asn Leu Thr Ala Gly Met Tyr Cys Ala Ala
           35                  40                  45
   Leu Glu Ser Leu Ile Asn Val Ser Gly Cys Ser Ala Ile Glu Lys Thr
       50                  55                  60
   Gln Arg Met Leu Ser Gly Phe Cys Pro His Lys Val Ser Ala Gly Gln
   65                  70                  75                  80
   Phe Ser Ser Leu His Val Arg Asp Thr Lys Ile Glu Val Ala Gln Phe
                   85                  90                  95
   Val Lys Asp Leu Leu Leu His Leu Lys Lys Leu Phe Arg Glu Gly Arg
               100                 105                 110
   Phe Asn
<210> 5
   <211> 114
   <212> PRT
   <213> Artificial Sequence
<220>
   <223> Description of Artificial Sequence: Synthetic
<400> 5
   Ser Pro Gly Pro Val Pro Pro Ser Thr Ala Leu Arg Lys Leu Ile Glu
    1               5                  10                  15
   Glu Leu Val Asn Ile Thr Gln Asn Gln Lys Ala Pro Leu Cys Asn Gly
               20                  25                  30
   Ser Met Val Trp Ser Ile Asn Leu Thr Ala Gly Met Tyr Cys Ala Ala
           35                  40                  45
   Leu Glu Ser Leu Ile Asn Val Ser Gly Cys Ser Ala Ile Glu Lys Thr
       50                  55                  60
   Gln Arg Met Leu Ser Gly Phe Cys Pro His Lys Val Ser Ala Gly Gln
   65                  70                  75                  80
   Phe Ser Ser Leu His Val Arg Asp Thr Lys Ile Glu Val Ala Gln Phe
                   85                  90                  95
   Val Lys Asp Leu Leu Leu His Leu Lys Lys Leu Phe Arg Glu Gly Arg
               100                 105                 110
   Phe Asn
<210> 6
   <211> 114
   <212> PRT
   <213> Artificial Sequence
<220>
   <223> Description of Artificial Sequence: Synthetic
<400> 6
   Ser Pro Gly Pro Val Pro Pro Ser Thr Ala Leu Arg Tyr Leu Ile Glu
    1               5                  10                  15
   Glu Leu Val Asn Ile Thr Gln Asn Gln Lys Ala Pro Leu Cys Asn Gly
               20                  25                  30
   Ser Met Val Trp Ser Ile Asn Leu Thr Ala Gly Met Tyr Cys Ala Ala
           35                  40                  45
   Leu Glu Ser Leu Ile Asn Val Ser Gly Cys Ser Ala Ile Glu Lys Thr
       50                  55                  60
   Gln Arg Met Leu Ser Gly Phe Cys Pro His Lys Val Ser Ala Gly Gln
   65                  70                  75                  80
   Phe Ser Ser Leu His Val Arg Asp Thr Lys Ile Glu Val Ala Gln Phe
                   85                  90                  95
   Val Lys Asp Leu Leu Leu His Leu Lys Lys Leu Phe Arg Glu Gly Arg
               100                 105                 110
   Phe Asn
<210> 7
   <211> 1283
   <212> DNA
   <213> Homo sapiens
<400> 7
   aagccaccca gcctatgcat ccgctcctca atcctctcct gttggcactg ggcctcatgg 60
   cgcttttgtt gaccacggtc attgctctca cttgccttgg cggctttgcc tccccaggcc 120
   ctgtgcctcc ctctacagcc ctcagggagc tcattgagga gctggtcaac atcacccaga 180
   accagaaggc tccgctctgc aatggcagca tggtatggag catcaacctg acagctggca 240
   tgtactgtgc agccctggaa tccctgatca acgtgtcagg ctgcagtgcc atcgagaaga 300
   cccagaggat gctgagcgga ttctgcccgc acaaggtctc agctgggcag ttttccagct 360
   tgcatgtccg agacaccaaa atcgaggtgg cccagtttgt aaaggacctg ctcttacatt 420
   taaagaaact ttttcgcgag ggacggttca actgaaactt cgaaagcatc attatttgca 480
   gagacaggac ctgactattg aagttgcaga ttcatttttc tttctgatgt caaaaatgtc 540
   ttgggtaggc gggaaggagg gttagggagg ggtaaaattc cttagcttag acctcagcct 600
   gtgctgcccg tcttcagcct agccgacctc agccttcccc ttgcccaggg ctcagcctgg 660
   tgggcctcct ctgtccaggg ccctgagctc ggtggaccca gggatgacat gtccctacac 720
   ccctcccctg ccctagagca cactgtagca ttacagtggg tgcccccctt gccagacatg 780
   tggtgggaca gggacccact tcacacacag gcaactgagg cagacagcag ctcaggcaca 840
   cttcttcttg gtcttattta ttattgtgtg ttatttaaat gagtgtgttt gtcaccgttg 900
   gggattgggg aagactgtgg ctgctggcac ttggagccaa gggttcagag actcagggcc 960
   ccagcactaa agcagtggac cccaggagtc cctggtaata agtactgtgt acagaattct 1020
   gctacctcac tggggtcctg gggcctcgga gcctcatccg aggcagggtc aggagagggg 1080
   cagaacagcc gctcctgtct gccagccagc agccagctct cagccaacga gtaatttatt 1140
   gtttttcctc gtatttaaat attaaatatg ttagcaaaga gttaatatat agaagggtac 1200
   cttgaacact gggggagggg acattgaaca agttgtttca ttgactatca aactgaagcc 1260
   agaaataaag ttggtgacag ata                                         1283
<210> 8
   <211> 18
   <212> PRT
   <213> Artificial Sequence
<220>
   <223> Description of Artificial Sequence: Synthetic
<400> 8
   Met Leu Ser Leu Arg Gln Ser Ile Arg Phe Phe Lys Pro Ala Thr Arg
   1 5 10 15
   Thr Leu
<210> 9
   <211> 8
   <212> PRT
   <213> Artificial Sequence
<220>
   <223> Description of Artificial Sequence: Synthetic
<400> 9
   Pro Pro Lys Lys Lys Arg Lys Val
   1 5

## Description

### FIELD OF THE INVENTION

The present invention is related to cell or pathogen destruction via fiberless radiative effectors that encapsulate a free radical generator.

### BACKGROUND OF THE INVENTION

Despite the enormous efforts and resources directed at finding a cure, cancer remains an elusive and deadly foe for mankind. In the United States, more than 900,000 new cases of cancer are diagnosed each year, and more than 600,000 people die from cancer every year. The standard methods of treatment usually include chemotherapy, radiation treatment, and surgical removal of tumors and/or growths, or some combination thereof. These treatments, combined with an emphasis on preventative lifestyle modification, have afforded a measure of success in the battle against some cancers. However, cancer remains one of the leading causes of mortality, and cancers detected at matured stages are typically fatal.

Numerous chemical agents have been devised for the treatment of cancer with varying degrees of efficacy. However, no single drug has one hundred percent effectiveness against different cancers, and negative side-effects ranging from minor to serious are always present.

One class of chemical agents that has found some use against cancer are photosensitizers. Such compounds are used for photodynamic therapy. Photodynamic therapy requires that the photosensitizer accumulate in the tumor. Local activation of the photosensitizer is accomplished by delivery of visible light to the tumor, generally via a laser. The primary mechanism of action is the *in situ* generation of an active form of molecular oxygen (singlet oxygen) that causes vascular stasis followed by vascular hemorrhage and tumor wall destruction. This process appears to be mediated by cytokines including prostaglandins, lymphokines, and thromboxanes. For example, the photosensitizer Photofrin II (porfimer sodium) has been used for the treatment of bladder cancer, endobronchial non-small cell cancer, retroperitoneal sarcoma, and malignant dysphagia in esophogeal cancer (*See, e.g.,* Dougherty and Marcus, Eur. J. Cancer, 28A(10):1734-42 [1992]).

Conventional photodynamic therapy has several significant limitations. Photofrin and other photosensitizers are normally given intravenously and do not specifically target tumors. Malignant and transformed cells do not demonstrate more selective or higher drug uptake than their normal counterparts. Therefore, the light source must be directed to the tumor and not surrounding tissue, which may also be damaged. Packaging Photofrin in liposomes (Jiang *et al*., Photochem. Photobiol., 67:23S [1998]) does not improve selectivity because the liposomes bind to and deposit Photofrin in normal cells as well. Furthermore, systemic administration of photosensitizers causes cutaneous photosensitivity that can last from six to eight weeks, and may lead to the development of contracted bladder due nonselective accumulation (Xioa *et al.,* Photochem. Photobiol. 67(5):573-83 [1998]). Attempts to solve these problems by utilizing intravesical (*i.e*., i.b.) administration have had mixed results. Also, because of the nonselectivity of current photosensitizers, relativity large amounts must be administered, resulting in a low therapeutic index. Finally, the use of photodyanamic therapy for some tumors is limited because of the inability to deliver light to the tumors.

Clearly, there is need for alternative strategies and reagents for photodynamic therapy. It would be desirable if such reagents could be administered in low doses, so that collateral damage of adjacent tissues or cells is low. It would also be desirable if the reagents and methods did not cause cutaneous photosensitivity.

### SUMMARY OF THE INVENTION

The present invention is related to pathogen and cancer cell destruction via fiberless radiative effectors that encapsulate a free radical generator or toxic agent release system. Such effectors find use *in vivo* and *in vitro.*

In particular, the present invention is related to a composition comprising a molecular recognition element attached to a fiberless radiative effector comprising a polymer, characterized in that said molecular recognition element is selected from the group consisting of a peptide, protein, carbohydrate, nucleic acid and biotin capable of binding to a target, said target being selected from the group consisting of a cell surface protein, a cell surface receptor, an extracellular matrix protein, a viral coat protein, and a bacterial cell wall protein, said polymer incorporating an agent, wherein said agent produces a toxic diffusable substance upon stimulation from an energy source.

In some embodiments, the present invention provides a fiberless radiative effector that has molecular recognition elements fixed thereto. In certain embodiments of the present invention, the fiberless radiative effector is toxic to or includes a system for generating molecules toxic to living cells.

A variety of compositions are contemplated. The fiberless radiative effectors of the present invention are preferably either solid or semisolid particles (*e.g*., nanospheres) preferably ranging in size between approximately 10 micrometers and 0.1 nanometers, and more preferably, between approximately 5 micrometers and 1 nanometer in diameter. In some embodiments of the present invention, the ultimate size of the fiberless radiative effectors is attained by fine grinding and filtering or by micro/nano-emulsion techniques used to form mono-disperse colloidal particles (*i.e,* rather than nano-fabrication). In some embodiments, the fiberless radiative effector is selected from polymer fiberless radiative effectors acrylamide fiberless radiative effectors, and sol-gel fiberless radiative effectors.

The fiberless radiative effectors of the present invention may be chosen from a variety of polymers. In some embodiments of the present invention, the polymer is selected from poly(vinyl chloride), poly(vinyl chloride) carboxylated, and poly(vinyl chloride-co-vinyl acetate-co-vinyl alcohol). In some preferred embodiments, the fiberless radiative effectors further comprise an additive and a plasticizer. In other embodiments, the present invention provides acrylamide radiative fiberless effectors comprising N,N-methylene-bi(acrylamide) polymerized into a gel.

Different molecular recognition elements are contemplated. In some embodiments, the molecular recognition element is a carbohydrate or a carbohydrate-binding protein (*e.g.,* a lectin). In some embodiments of the present invention, the molecular recognition element is a polypeptide. In some embodiments of the present invention, the polypeptide is preferably an antigen binding protein. In further embodiments of the present invention, the antigen binding protein is selected from antibodies, humanized antibodies, F(ab) fragments, F(ab)₂ fragments, and single chain antibodies. In other embodiments of the present invention, the polypeptide is preferably an agonist or antagonist that binds to a cell surface receptor. In still other embodiments of the present invention, the polypeptide is preferably avidin. In still other embodiments of the present invention, the polypeptide is preferably a signal sequence that directs the fiberless radiative effector to an intracellular compartment (*e.g*., mitochondria or nucleus). In some embodiments of the present invention, the molecular recognition elements comprises a nucleic acid. In some preferred embodiments, the nucleic acid is selected from RNA and DNA. In still further embodiments of the present invention, the molecular recognition element is preferably an organic molecule. In some embodiments, the organic molecule is preferably biotin. Different toxicities and systems for generating toxic molecules (*e.g.,* singlet oxygen, hydroxyl radicals, and superoxide radicals or nitric oxide) are contemplated. In some embodiments, the fiberless radiative effector further comprises a system for generating toxic free radicals, preferably oxygen or nitrogen free radicals. In further embodiments, the system for generating free radicals comprises a photodynamic dye. In some embodiments, the photodynamic compound are selected from Photofrin, ruthenium red compounds (*i.e.,* Ru-diphenyl-phenanthroline and Tris(1-10-phenanthroline)ruthenium(II) chloride), tin ethyl etiopurpurin, protoporphyrin IX, chloroaluminum phthalocyanine, tetra(M-hydroxyphenyl)chlorin.

In other embodiments of the present invention, the system for generating free radicals comprises a radiodynamic (i.e., scintillating) compound. In some embodiments, the radiodynamic compounds are selected from NaI-125, 2,5-diphenyloxazole (PPO); 2-(4-biphenyl)-6-phenylbenzoxazole; 2,5-bis-(5'-tert-butylbenzoxazoyl-[2'])thiophene; 2-(4-t-butylphenyl)-5-(4-biphenylyl)-1,3,4-oxadiazole; 1,6-diphenyl-1,3,5-hexatriene; trans-p,p'-diphenylstilbene; 2-(1-naphthyl)-5-phenyloxazole; 2-phenyl-5-(4-biphenylyl)-1,3,4-oxadiazole; p-terphenyl; and 1,1,4,4-tetraphenyl-1,3-butadiene. In some embodiments, the radiodynamic system for generating toxic materials further comprises a supra- or super-molecular antenna system (e.g., styrene, polystyrene, or poly(styrene)-poly(vinylpyridine). In other embodiments, the radiodynamic system for generating toxic materials further comprises a photodynamic compound (*e.g*., porphyrin, Photofrin, hematoporphyrin).

In some embodiments of the present invention, the fiberless radiative effector further comprises a radioactive element. Radioactive elements may be selected from elements or compounds that emit gamma rays (*e.g.*, ¹¹¹In-oxine, ⁵⁹Fe, ⁶⁷Cu, ¹²⁵I, and ⁵¹Cr) or compounds that emit beta particles (*e.g.,* ³²P, ³H, ³⁵S, ¹⁴C).

In some embodiments, the fiberless radiative effector further comprises a cloaking agent or means. In some embodiments, the cloaking agent or means is a liposome in which the fiberless effectors are packaged. In other embodiments, the cloaking agent or means is a red blood cell in which the fiberless effectors are packaged. In still further embodiments, the cloaking agent or means is preferably a cloaking film. A variety of cloaking agents are contemplated, including the following: poly(ethylene glycol), poly(acrylamide), poly(vinyl pyrrolidone), poly(ethylene glycol)-phosphatidyl.

The fiberless radiative effectors of the present invention are utilized to destroy target organisms, cells or proteins or other macromolecules. The target organism is preferably a prion or bacterial organism *(e.g., Legionella peomophilia, Mycobacterium tuberculosis, Clostridium tetani, Hemophilus influenzae, Neisseria gonorrhoeae, Treponema pallidum, Bacillus anthracis, Vibrio cholerae, Borrelia burgdorferi, Cornebacterium diphtheria* or *Staphylococcus aureus* ). In other embodiments, the target organism is preferably a virus (*e.g.,* human papilloma virus, human immunodeficiency virus, rubella virus, polio virus). In some embodiments, the target cells are cancer cells selected from topical cells *(e.g,* malignant melanoma cells and basal cell carcinomas), ductal cells (*e.g.,* mammary ductal adenocarcinoma cell and bowel cancer cells), and deep tissue cells (*e.g.,* hepatocellular carcinoma cells, CNS primary lymphoma cells, and glioma cells). In some embodiments, the target cell or organism includes a target epitope. In some embodiments, the target epitope is selected from cell surface proteins, cell surface receptors, cell surface polysaccharides, extracellular matrix proteins, intracellular proteins and intracellular nucleic acids. In still other embodiments, the effector is targeted via a signal peptide to a particular cellular organelle (*e.g*., mitochondria or the nucleus).

In some embodiments, the fiberless radiative effector binds to a cell surface protein, a cell surface receptor, a extracellular matrix protein, a viral coat protein, a bacterial cell wall protein, a viral or bacterial polysaccharide, or be directed to a particular organelle within a cell. In some embodiments of the present invention, the fiberless radiative effector further comprises a photodynamic dye. In still further embodiments, the first effector-target complex is exposed to light so that singlet oxygen is produced. The singlet oxygen causes oxidation and necrosis/apoptosis of the target cell, macromolecule, or organism.

In some embodiments, the present invention comprises providing a first fiberless radiative effector comprising a first molecular recognition element and a stacking epitope, a second fiberless radiative effector comprising a second molecular recognition element, and a biological target comprising a target epitope. In further embodiments of the present invention, one of the first and second fiberless radiative effectors further comprises a radiodynamic or photodynamic dye, while the other of the first and second radiative fiberless effectors comprises a radioactive element that emits energy (*e.g*., alpha particles, beta particles, or gamma rays). In some embodiments, the release of alpha or beta particles or gamma rays by the radioactive element causes the excitation of the radiodynamic or photodynamic dye, causing the production of singlet oxygen or oxygen free radicals. In some embodiments of the present invention, the biological target, first fiberless radiative effector and second fiberless radiative effector are combined so that the first fiberless radiative effector binds to the target epitope on the biological target and the second fiberless radiative effector binds to the stacking epitope on the first fiberless radiative effector. The binding of the first fiberless radiative effector to the second radiative fiberless effector brings the radioactive element into close proximity with the radiodynamic or photodynamic dye, resulting in the production of singlet oxygen which causes the oxidation of macromolecules and necrosis/apoptosis of the target cell.

In still another embodiment, the present invention provides an alternative stacking approach. This approach allows a universal fiberless radiative effector (*e.g.*, a fiberless radiative effector with either avidin or biotin as its molecular recognition element) to be used to destroy a variety of biological targets. In some embodiments, the present invention comprises providing a first fiberless radiative effector comprising a first molecular recognition element (*e.g*., avidin), a biological target comprising a target epitope, and a stacking reagent (*e.g.*, a biotinylated antigen binding protein) comprising a molecular recognition element and at least one stacking epitope. In some embodiments of the present invention, the first fiberless radiative effector comprises a photodynamic or radiodynamic dye. In other embodiments, a second fiberless radiative effector is provided that comprises a radioactive element that emits energy. In some embodiments of the present invention, the target cell, stacking reagent and first fiberless radiative effector are combined so that the stacking reagent molecular recognition element binds to the biological target and the fiberless radiative effector molecular recognition element binds to the stacking reagent epitope to form a target cell-stacking reagent-fiberless radiative effector complex. In some embodiments, the complex is exposed to a light source that excites the photodynamic dye so that singlet oxygen is produced which causes oxidation and necrosis of the target cell. In other embodiments, a second fiberless radiative effector comprising a radioactive element and a second molecular recognition element is provided. The second molecular recognition element preferably binds to a second stacking epitope on the stacking reagent so that a complex is formed. In some preferred embodiments, the alpha or beta particles emitted by the radioactive element in the second fiberless radiative effector excite the radiodynamic or photodynamic dye in the first fiberless radiative effector so that singlet oxygen is produced.

In some embodiments, a fiberless radiative effector comprising a fluorescent photosensitizer or dye and a molecular recognition element and a biological target are provided. In some embodiments, the fiberless radiative effector and biological target are combined so that the molecular recognition element binds to the biological target to form a target-fiberless radiative effector complex. In other embodiments, the presence of the fiberless radiative effector is detected by exposing the complex to light of the appropriate wavelength that causes the photosensitizer or dye to fluoresce. In still further embodiments, fluorescence is detected by a luminometer, by a microscope equipped for epifluorescence, or by capturing a digital image from a microscope with a CCD camera. In still further embodiments, the fluorescent signal is quantitated by determining pixel intensity.

### DESCRIPTION OF THE DRAWINGS

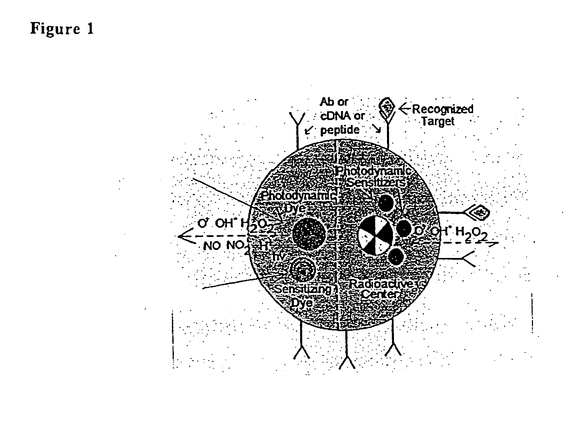

Fig. 1 is a schematic composite (photodynamic/radiodynamic) depiction of a fiberless radiative effector.
Fig. 2 is a photomicrograph of cells that were lipofected with fiberless radiative effectors.
Fig. 3 is a photomicrograph demonstrating lipofection with PEBBLEs.
Fig. 4 depicts plots of tumor volumes obtained form serial MRI versus time following 9L cell implantation.
Fig. 5 lists the sequences for (A) wild-type IL13 cDNA, (B) wild-type IL13 protein, and the protein sequences for the 1113 variants (C) hIL13.E13K and (D) hIL13.E13Y.

### DEFINITIONS

To facilitate understanding of the invention, a number of terms are defined below.

As used herein, the term "fiberless radiative effector" refers to a polymeric particle *(i.e.,* a particle as opposed to a fiber) or particle comprising a porous inorganic glass *(e.g.,* sol-gel) that is light penetrable (*i.e*., by photons) or penetrable to some other energy source (*e.g.,* α or β particles or gamma rays) and contains a toxic agent. The terms "core" and "core matrix" when used in reference to fiberless radiative effectors refer to the polymeric or porous metal oxide portion of the fiberless radiative effector.

As used herein, the term "nanosphere" refers to a polymeric or sol-gel particle of about approximately 0.5 nanometers to 10 micrometers in diameter.

As used herein, the term "molecular recognition element" refers to molecular capable of binding to, hybridizing to, or otherwise interacting with another molecule. Examples of molecular recognition elements include nucleic acid molecules (*e.g.,* RNA and DNA, including ligand-binding RNA molecules), polypeptides (*e.g.*, antigen binding proteins, receptor ligands, signal peptides, hydrophobic membrane spanning domains), and organic molecules (*e.g*., biotin). A given fiberless radiative effector may have affixed thereto one or a variety of molecular recognition elements.

As used herein, the term "toxic agent" refers to a material or mixture of materials which are themselves toxic to a biological system (*e.g.*, pathogen, virus, bacteria, cell, or multicellular organism) or which upon a stimulus (*e.g*., light, α or β particles) produce an agent (*e.g*., singlet oxygen or free radical) which is toxic to a biological system.

As used herein, the terms "cloaking material" and "cloaking agent" refers to any material used to protect a fiberless radiative effector from recognition by the reticulo-endothelial system, including poly(ethylene glycol) and liposomes.

As used herein, the term "stacking epitope" refers to a material (*e.g.,* biotin) on a first fiberless radiative effector that is recognized by the molecular recognition element of a second fiberless radiative effector or some other material (e.g., avidin or antigen binding protein).

As used herein, the term "stacking reagent" refers to a material that binds to a biological target and is recognizable by the molecular recognition element of a fiberless radiative effector.

As used herein, the term "stacking reagent epitope" refers to a material (*e.g.,* biotin or avidin) on a stacking reagent that is recognizable be a molecular recognition element.

As used herein, the term "antigen binding protein" refers to proteins which bind a specific antigen. "Antigen binding proteins" include immunoglobulin, polyclonal, monoclonal, chimeric, single chain, and humanized antibodies, Fab fragments, F(ab')2 fragments, and Fab expression libraries.

As used herein the term "biological target" refers to any organism, cell, microorganism, bacteria, virus, fungus, plant, prion, protozoa, or pathogen or portion of an organsim, cell, microorganism, bacteria, virus, fungus, plant, prion, protozoa or pathogen.

As used herein, the terms "peptide" or "polypeptide" refer to a chain of amino acids (i.e., two or more amino acids) linked through peptide bonds between the α-carboxyl carbon of one amino acid residue and the α-nitrogen of the next. A "peptide" or "polypeptide" may comprise an entire protein or a portion of protein. "Peptides" and "polypeptides" may be produced by a variety of methods including chemical synthesis, translation from a messenger RNA, expression in a host cell, expression in a cell free translation system, and digestion of another polypeptide.

As used herein the term "protein" is used in its broadest sense to refer to all molecules or molecular assemblies containing two or more amino acids. Such molecules include proteins, peptides, enzymes, antibodies, receptors, lipoproteins, and glycoproteins.

As used herein, the term "enzyme" refers to molecules or molecule aggregates that are responsible for catalyzing chemical and biological reactions. Such molecules are typically proteins, but can also comprise short peptides, RNAs, ribozymes and antibodies.

As used herein, the terms "nucleic acid" or "nucleic acid molecules" refer to any nucleic acid containing molecule including DNA or RNA. The term encompasses sequences that include any of the known base analogs of DNA and RNA including 4-acetylcytosine, 8-hydroxy-N6-methyladenosine, aziridinylcytosine, pseudoisocytosine, 5-(carboxyhydroxylmethyl) uracil, 5-fluorouracil, 5-bromouracil, 5-carboxymethylaminomethyl-2-thiouracil, 5-carboxymethylaminomethyluracil, dihydrouracil, inosine, N6-isopentenyladenine, 1-methyladenine, 1-methylpseudouracil, 1-methylguanine, 1-methylinosine, 2,2-dimethylguanine, 2-methyladenine, 2-methylguanine, 3-methylcytosine, 5-methylcytosine, N6-methyladenine, 7-methylguanine, 5-methylaminomethyluracil, 5-methoxyaminomethyl-2-thiouracil, beta-D-mannosylqueosine, 5'-methoxycarbonylmethyluracil, 5-methoxyuracil, 2-meihylthio-N6-isopentenyladenine, uracil-5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, N-uracil-5-oxyacetic acid methylester, uracil-5-oxyacetic acid, pseudouracil, queosine, 2-thiocytosine, and 2,6-diaminopurine.

Nucleic acid molecules are said to have "5' ends" and "3' ends" because mononucleotides are reacted to make oligonucleotides or polynucleotides in a manner such that the 5' phosphate of one mononucleotide pentose ring is attached to the 3' oxygen of its neighbor in one direction via a phosphodiester linkage. Therefore, an end of an oligonucleotides or polynucleotide, referred to as the "5' end" if its 5' phosphate is not linked to the 3' oxygen of a mononucleotide pentose ring and as the "3' end" if its 3' oxygen is not linked to a 5' phosphate of a subsequent mononucleotide pentose ring. As used herein, a nucleic acid sequence, even if internal to a larger oligonucleotide or polynucleotide, also may be said to have 5' and 3' ends. In either a linear or circular DNA molecule, discrete elements are referred to as being "upstream" or 5' of the "downstream" or 3' elements. This terminology reflects the fact that transcription proceeds in a 5' to 3' fashion along the DNA strand. The promoter and enhancer elements that direct transcription of a linked gene are generally located 5' or upstream of the coding region. However, enhancer elements can exert their effect even when located 3' of the promoter element and the coding region. Transcription termination and polyadenylation signals are located 3' or downstream of the coding region.

As used herein, the terms "complementary" or "complementarity" are used in reference to polynucleotides (*i.e.,* a sequence of nucleotides) related by the base-pairing rules. For example, for the sequence "A-G-T," is complementary to the sequence "T-C-A." Complementarity may be "partial," in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridization between nucleic acid strands. This is of particular importance in amplification reactions, as well as detection methods that depend upon binding between nucleic acids.

The term "homology" refers to a degree of complementarity. There may be partial homology or complete homology (*i.e*., identity). A partially complementary sequence is one that at least partially inhibits a completely complementary sequence from hybridizing to a target nucleic acid and is referred to using the functional term "substantially homologous." The inhibition of hybridization of the completely complementary sequence to the target sequence may be examined using a hybridization assay (Southern or Northern blot, solution hybridization) under conditions of low stringency. A substantially homologous sequence or probe will compete for and inhibit the binding (*i.e.,* the hybridization) of a completely homologous to a target under conditions of low stringency. This is not to say that conditions of low stringency are such that non-specific binding is permitted; low stringency conditions require that the binding of two sequences to one another be a specific *(i.e.,* selective) interaction. The absence of non-specific binding may be tested by the use of a second target that lacks even a partial degree of complementarity (*e.g.*, less than about 30% identity); in the absence of non-specific binding the probe will not hybridize to the second non-complementary target.

The art knows well that numerous equivalent conditions may be employed to comprise low stringency conditions; factors such as the length and nature (DNA, RNA, base composition) of the probe and nature of the target (DNA, RNA, base composition, present in solution or immobilized, etc.) and the concentration of the salts and other components (*e.g*., the presence or absence of formamide, dextran sulfate, polyethylene glycol) are considered and the hybridization solution may be varied to generate conditions of low stringency hybridization different from, but equivalent to, the above listed conditions. In addition, the art knows conditions that promote hybridization under conditions of high stringency (*e.g.,* increasing the temperature of the hybridization and/or wash steps, the use of formamide in the hybridization solution, etc.).

When used in reference to a double-stranded nucleic acid sequence such as a cDNA to an RNA of the present invention, the term "substantially homologous" refers to any probe that can hybridize to either or both strands of the double-stranded nucleic acid sequence under conditions of low stringency as described above.

When used in reference to a single-stranded nucleic acid sequence, the term "substantially homologous" refers to any probe that can hybridize (*i.e*., it is the complement of) the single-stranded nucleic acid sequence under conditions of low stringency as described above.

As used herein, the term "hybridization" is used in reference to the pairing of complementary nucleic acids. Hybridization and the strength of hybridization (*i.e*., the strength of the association between the nucleic acids) is impacted by such factors as the degree of complementary between the nucleic acids, stringency of the conditions involved, the Tₘ of the formed hybrid, and the G:C ratio within the nucleic acids.

As used herein, the term "Tₘ" is used in reference to the "melting temperature." The melting temperature is the temperature at which a population of double-stranded nucleic acid molecules becomes half dissociated into single strands. The equation for calculating the Tₘ of nucleic acids is well known in the art. As indicated by standard references, a simple estimate of the Tₘ value may be calculated by the equation: Tₘ = 81.5 + 0.41(% G + C), when a nucleic acid is in aqueous solution at 1 M NaCl (*See e.g.,* Anderson and Young, Quantitative Filter Hybridization, *in Nucleic Acid Hybridization* [1985]). Other references include more sophisticated computations that take structural as well as sequence characteristics into account for the calculation of Tₘ.

As used herein the term "stringency" is used in reference to the conditions of temperature, ionic strength, and the presence of other compounds such as organic solvents, under which nucleic acid hybridizations are conducted. Those skilled in the art will recognize that "stringency" conditions may be altered by varying the parameters just described either individually or in concert. With "high stringency" conditions, nucleic acid base pairing will occur only between nucleic acid fragments that have a high frequency of complementary base sequences (*e.g*., hybridization under "high stringency" conditions may occur between homologs with about 85-100% identity, preferably about 70-100% identity). With medium stringency conditions, nucleic acid base pairing will occur between nucleic acids with an intermediate frequency of complementary base sequences (e.g., hybridization under "medium stringency" conditions may occur between homologs with about 50-70% identity). Thus, conditions of "weak" or "low" stringency are often required with nucleic acids that are derived from organisms that are genetically diverse, as the frequency of complementary sequences is usually less.

As used herein, the term "oligonucleotide," refers to a short length of single-stranded polynucleotide chain. Oligonucleotides are typically less than 100 residues long (*e*.*g*., between 15 and 50), however, as used herein, the term is also intended to encompass longer polynucleotide chains. Oligonucleotides are often referred to by their length. For example a 24 residue oligonucleotide is referred to as a "24-mer". Oligonucleotides can form secondary and tertiary structures by self-hybridizing or by hybridizing to other polynucleotides. Such structures can include duplexes, hairpins, cruciforms, bends, and triplexes.

As used herein, the term "polymer" refers to material comprised of repeating subunits. Examples of polymers include polyacrylamide and poly(vinyl chloride), poly(vinyl chloride) carboxylated, and poly(vinyl chloride-co-vinyl acetate co-vinyl) alcohols.

As used herein, the term "polymerization" encompasses any process that results in the conversion of small molecular monomers into larger molecules consisting of repeated units. Typically, polymerization involves chemical crosslinking of monomers to one another.

As used herein, the term "plasticizer" refers to organic compounds added to a polymer to modify it by internal modification (i.e., solvation). The plasticizer replaces some secondary valence bonds between polymer subunits with plasticizer-to-polymer bonds. Examples of plasticizers include benzyl ether, benzyl 2-nitrophenyl ether, 1-decanol, 2-nitrodiphenyl ether, 1-octodecanol.

As used herein, the terms "photosensitizer," and "photodynamic dye," refer to materials which undergo transformation to an excited state upon exposure to a light quantum (hv). Examples of photosensitizers and photodynamic dyes include, Photofrin 2, benzoporphyrin, m-tetrahydroxyphenylchlorin, tin etiopurpurin, copper benzochlorin, and other porphyrins.

As used herein, the term "radiodynamic compound" refers to materials which undergo a transition an excited state upon exposure to x-rays, gamma ray, or beta particles. Examples of radiodynamic compounds include polystyrene and scintillating compounds (*e.g.,* NaI-125, 2,5-diphenyloxazole (PPO); 2-(4-biphenyl)-6-phenylbenzoxazole; 2,5-bis-(5'-tert-butylbenzoxazoyl-[2'])thiophene; 2-(4-t-butylphenyl)-5-(4-biphenylyl)-1,3,4-oxadiazole; 1,6-diphenyl-1,3,5-hexatriene; trans-p,p'-diphenylstilbene; 2-(1-naphthyl)-5-phenyloxazole; 2-phenyl-5-(4-biphenylyl)-1,3,4-oxadiazole; p-terphenyl; and 1,1,4,4-tetraphenyl-1,3-butadiene)

As used herein, the term "radioactive element" refers to any material which emits α, β, or γ particles.

As used herein, the term "biological target" refers to any cell, bacterium, virus, or portion thereof, including, cell walls, cell surface receptors, extracellular matrix proteins, viral coat proteins, nucleic acids (*e.g*., RNA and DNA), proteins, and organelles (*e.g*, mitochondria and nuclei).

As used herein, the term "target-fiberless radiative effector complex" or simple "target-effector complex" refers to at least one fiberless radiative effector bound to, contained in, or otherwise affixed to a biological target.

As used herein, the term "reaction" refers to any change or transformation in which a substance (*e.g*., molecules, membranes, and molecular assemblies) combines with other substances, interchanges constituents with other substances, decomposes, rearranges, or is otherwise chemically altered. As used herein, the term "reaction means" refers to any means of initiating and/or catalyzing a reaction. Such reaction means include, enzymes, temperature changes, and pH changes. The phrase "affinity for said reaction means" refers to compounds with the ability to specifically associate (*e.g*., bind) to a given reaction mean, although not necessarily a substrate for the reaction means. For example, a PLA₂ antibody has affinity for PLA₂, but is not the substrate for the enzyme.

As used herein, the term "immobilization" refers to the attachment or entrapment, either chemically or otherwise, of material to another entity (*e.g*., a solid support) in a manner that restricts the movement of the material.

As used herein, the terms "material" and "materials" refer to, in their broadest sense, any composition of matter.

As used herein, the term "antigen" refers to any molecule or molecular group that is recognized by at least one antibody. By definition, an antigen must contain at least one epitope (*i.e*., the specific biochemical unit capable of being recognized by the antibody). The term "immunogen" refers to any molecule, compound, or aggregate that induces the production of antibodies. By definition, an immunogen must contain at least one epitope.

As used herein the term "antibody" refers to a glycoprotein evoked in an animal by an immunogen (antigen). An antibody demonstrates specificity to the immunogen, or, more specifically, to one or more epitopes contained in the immunogen. Native antibody comprises at least two light polypeptide chains and at least two heavy polypeptide chains. Each of the heavy and light polypeptide chains contains at the amino terminal portion of the polypeptide chain a variable region (*i.e.,* VH and VL respectively), which contains a binding domain that interacts with antigen. Each of the heavy and light polypeptide chains also comprises a constant region of the polypeptide chains (generally the carboxy terminal portion) which may mediate the binding of the immunoglobulin to host tissues or factors influencing various cells of the immune system, some phagocytic cells and the first component (Clq) of the classical complement system. The constant region of the light chains is referred to as the "CL region," and the constant region of the heavy chain is referred to as the "CH region." The constant region of the heavy chain comprises a CH1 region, a CH2 region, and a CH3 region. A portion of the heavy chain between the CH1 and CH2 regions is referred to as the hinge region (i.e., the "H region"). The constant region of the heavy chain of the cell surface form of an antibody further comprises a spacer-transmembranal region (M1) and a cytoplasmic region (M2) of the membrane carboxy terminus. The secreted form of an antibody generally lacks the M1 and M2 regions.

As used herein, the term "sol-gel" refers to preparations composed of porous metal oxide glass structures. Such structures can have biological material entrapped within the porous structures. The phrase "sol-gel matrices" refers to the structures comprising the porous metal oxide glass with or without entrapped material. The term "sol-gel material" refers to any material prepared by the sol-gel process including the glass material itself and any entrapped material within the porous structure of the glass. As used herein, the term "sol-gel method" refers to any method that results in the production of porous metal oxide glass. In some embodiments, "sol-gel method" refers to such methods conducted under mild temperature conditions. The terms "sol-gel glass" and "metal oxide glass" refer to glass material prepared by the sol-gel method and include inorganic material or mixed organic/inorganic material. The materials used to produce the glass can include aluminates, aluminosilicates, titanates and ormosils and (organically modified silanes).

As used herein, the term "selective binding" refers to the binding of one material to another in a manner dependent upon the presence of a particular molecular structure (*i.e*., specific binding). For example, a receptor will selectively bind ligands that contain the chemical structures complementary to the ligand binding site(s). This is in contrast to "nonselective binding" or "background binding," whereby interactions are arbitrary and not based on structural compatibilities of the molecules.

As used herein, the term "pathogen" refers to disease causing organisms, microorganisms, or agents including viruses, bacteria, parasites (including organisms within the phyla Protozoa, Platyhelminthes, Aschelminithes, Acanthocephala, and Arthropoda), fungi, and prions.

As used herein, the term "bacteria" and "bacterium" refer to all prokaryotic organisms, including those within all of the phyla in the Kingdom Procaryotae. It is intended that the term encompass all microorganisms considered to be bacteria including *Mycoplasma, Chlamydia, Actinomyces, Streptomyces,* and *Rickettsia.* All forms of bacteria are included within this definition including cocci, bacilli, spirochetes, spheroplasts, protoplasts "Gram negative" and "gram positive" refer to staining patterns obtained with the Gram-staining process which is well known in the art *(See e.g.,* Finegold and Martin, Diagnostic Microbiology, 6th Ed. (1982), CV Mosby St. Louis, pp 13-15).

As used herein, the term "macromolecule" refers to large molecules such as proteins, polysaccharides, nucleic acids, and multiple subunit proteins. Examples of macromolecules include verotoxin I, verotoxin II, Shiga-toxin, botulinum toxin, snake venoms, insect venoms, alpha-bungarotoxin, and tetrodotoxin).

As used herein, the term "virus" refers to infectious agents, which with certain exceptions, are not observable by light microscopy, lack independent metabolism, and are able to replicate only within a host cell. The individual particles (*i.e*., virions) consist of nucleic acid and a protein shell or coat; some virions also have a lipid containing membrane. The term "virus" encompasses all types of viruses, including animal, plant, phage, and other viruses.

As used herein, the term "membrane receptors" refers to constituents of membranes that are capable of interacting with other molecules or materials. Such constituents can include proteins, lipids, carbohydrates, and combinations thereof.

As used herein, the term "drug" refers to a substance or substances that are used to diagnose, treat, or prevent diseases or conditions. Drugs act by altering the physiology of a living organism, tissue, cell, or *in vitro* system that they are exposed to. It is intended that the term encompass antimicrobials, including antibacterial, antifungal, and antiviral compounds. It is also intended that the term encompass antibiotics, including naturally occurring, synthetic, and compounds produced by recombinant DNA technology.

As used herein, the term "carbohydrate" refers to a class of molecules including sugars, starches, cellulose, chitin, and glycogen. Carbohydrates can also exist as components of glycolipids and glycoproteins.

As used herein, the term "chelating compound" refers to any compound composed of or containing coordinate links that complete a closed ring structure. The compounds can combine with metal ions, attached by coordinate bonds to at least two of the nonmetal ions.

As used herein, the term "molecular recognition complex" refers to any molecule, molecular group, or molecular complex that is capable of recognizing (*i.e.*, specifically interacting with) a molecule. For example, the ligand binding site of a receptor would be considered a molecular recognition complex.

As used herein, the term "room temperature" refers, technically, to temperatures approximately between 20 and 25 degrees centigrade. However, as used generally, it refers to the any ambient temperature within a general area in which an experiment is taking place.

As used herein, the term "encapsulate" refers to the process of encompassing, encasing, or otherwise associating two or more materials such that the encapsulated material is contained and/or immobilized within or onto the encapsulating material.

As used herein, the term "optical transparency" refers to the property of matter whereby the matter is capable of transmitting light such that the light can be observed by visual light detectors (*e.g*., CCD, photomultipliers, or other detection equipment).

As used herein, the term "organic solvents" refers to any organic molecules capable of dissolving another substance. Examples include chloroform, alcohols, phenols, and ethers.

As used herein, term "nanostructures" refers to microscopic structures, typically measured on a nanometer scale. Such structures include various three-dimensional assemblies, including liposomes, films, multilayers, braided, lamellar, helical, tubular, and fiber-like shapes, and combinations thereof. Such structures can, in some embodiments, exist as solvated polymers in aggregate forms such as rods and coils. The term "solvated polymers" refers to polymer nanostructures that are dissolved in a solvent.

As used herein, the term "liposome" refers to artificially produced spherical lipid complexes that can be induced to segregate out of aqueous media. The terms "liposome" and "vesicle" are used interchangeably herein.

As used herein, the term "biopolymeric liposomes" refers to liposomes that are composed entirely, or in part, of biopolymeric material.

As used herein, the term "ligands" refers to any ion, molecule, molecular group, or other substance that binds to another entity to form a larger complex. Examples of ligands include peptides, carbohydrates, nucleic acids (*e.g.*, DNA and RNA), antibodies

As used herein, the term "polymeric matrix" refers to matrices whereby some or all of the molecular constituents of the matrix are polymerized.

As used herein, the terms "head group" and "head group functionality" refer to the molecular groups present an the ends of molecules (*e.g*., the primary amine group at the end of peptides).

As used herein, the term "hydrophilic head-group" refers to ends of molecules that are attracted to water by chemical interactions including, hydrogen-bonding, van der Waals' forces, ionic interactions, or covalent bonds. As used herein, the term "hydrophobic head-group" refers to ends of molecules that self-associate with other hydrophobic entities, resulting in repulsion from water.

As used herein, the term "carboxylic acid head groups" refers to organic compounds containing one or more carboxyl (-COOH) groups located at, or near, the end of a molecule. The term carboxylic acid includes carboxyl groups that are either free or exist as salts or esters.

As used herein, the term "linker" or "spacer molecule" refers to material that links one entity to another. In one sense, a molecule or molecular group can be a linker that is covalently attached two or more other molecules (*e.g*., linking a ligand to a self-assembling monomer).

As used herein, the term "bond" refers to the linkage between atoms in molecules and between ions and molecules in crystals. The term "single bond" refers to a bond with two electrons occupying the bonding orbital. Single bonds between atoms in molecular notations are represented by a single line drawn between two atoms (*e.g.,* C₈-C₉). The term "double bond" refers to a bond that shares two electron pairs. Double bonds are stronger than single bonds and are more reactive. The term "triple bond" refers to the sharing of three electron pairs. As used herein, the term "ene-yne" refers to alternating double and triple bonds. As used herein the terms "amine bond," "thiol bond," and "aldehyde bond" refer to any bond formed between an amine group (*i.e*., a chemical group derived from ammonia by replacement of one or more of its hydrogen atoms by hydrocarbon groups), a thiol group (*i.e*., sulfur analogs of alcohols), and an aldehyde group (*i.e*., the chemical group -CHO joined directly onto another carbon atom), respectively, and another atom or molecule.

As used herein, the term "covalent bond" refers to the linkage of two atoms by the sharing of at least one electron, contributed by each of the atoms.

As used the term "absorption" refers, in one sense, to the absorption of light. Light is absorbed if it is not reflected from or transmitted through a sample. Samples that appear colored have selectively absorbed all wavelengths of white light except for those corresponding to the visible colors that are seen.

As used herein, the term "spectrum" refers to the distribution of electromagnetic energies arranged in order of wavelength.

As used herein, the term "magnetocatalysis" refers to cellular apoptosis resulting from exposure to a magnetic field.

As used the term "visible spectrum" refers to light radiation that contains wavelengths from approximately 360 nm to approximately 800 nm.

As used herein, the term "ultraviolet irradiation" refers to exposure to radiation with wavelengths less than that of visible light (*i.e*., less than approximately 360 nM) but greater than that of X-rays (*i.e.,* greater than approximately 0.1 nM). Ultraviolet radiation possesses greater energy than visible light and is therefore, more effective at inducing photochemical reactions.

As used herein, the term "infrared radiation" refers to exposure to radiation with wavelengths of greater 800 nM.

As used herein, the term "biological organisms" refers to any carbon-based life forms.

As used herein, the terms "signalling material" and "marker" refers to any material or molecule (*e.g*., luciferase, magnetic particle, or rhodamine) that is detectable by any detection system, including enzyme (*e.g*., ELISA, as well as enzyme-based histochemical assays), fluorescent, radioactive, nuclear magnetic resonance, and luminescent systems.

As used herein, the term "sample" is used in its broadest sense. In one sense, it is meant to include a specimen or culture obtained from biological and environmental samples. Biological samples may be obtained from animals (including humans) and encompass fluids, solids, tissues, and gases. Biological samples include blood products, such as plasma and serum, Environmental samples include environmental material such as surface matter, soil, water, crystals and industrial samples.

As used herein, the term "homobifunctional," refers to a linker molecule with two functional groups that both react with the same chemical group (*e.g*., primary amines, esters or aledehydes).

As used herein, the term "homobifunctional," refers to a linker molecule with two functional groups that react with different chemical groups (*e.g.,* primary amines, esters or aledehydes).

### DESCRIPTION OF THE INVENTION

The present invention is related to cell or pathogen destruction via fiberless radiative effectors that encapsulate a toxic agent or free radical generator.

The fiberless radiative or optical effectors of the present invention comprise one or more toxic agents embedded in a matrix material and a molecular recognition element for targeting the effector to a biological target. The matrix material takes many forms, including, polymeric matrices (*e.g*., poly(acrylamide), poly(vinyl chloride), decyl-methacrylate or poly(lactic acid)) or sol-gel matrices. In preferred embodiments, the fiberless radiative effector is generally spherical, however, the effector takes many shapes and sizes (*e.g*., helical, tubular, square, or rectangular). In some preferred embodiments, the fiberless radiative effector is about 40 to 400 nm in diameter. A variety of toxic agents are incorporated into the fiberless radiative effector matrix. In preferred embodiments, the toxic agents participate in the production of toxic diffusable molecules (*e.g*., singlet oxygen, hydroxyl radicals, or superoxide) upon stimulation or excitation from an energy source (*e.g*., incandescent light, red light, laser, x-rays, or gamma-rays).

A variety of molecular recognition elements are attached to the fiberless radiative effector or used to target the fiberless radiative effector to a biological target. Molecular recognition elements include antigen binding proteins, ligands for cell surface receptors, and nucleic acids. The molecular recognition elements are either attached, covalently or otherwise, to the fiberless radiative effector matrix, or it is attached to a cloaking agent that surrounds or coats the fiberless radiative effector. A variety of cloaking agents are utilized with the fiberless radiative effectors of the present invention, including poly(ethylene glycol), liposomes, poly(ethylene glycol) coated liposomes, and red blood cell ghosts.

The fiberless radiative effectors of the present invention have a number of uses. In some embodiments of the present invention, the fiberless radiative effectors are used to destroy or inhibit the growth of tumor cells. In some embodiments, the RGD peptide is used to target the fiberless radiative effector to the vasculature of head and neck tumors. In other embodiments, IL13 or one of its variants is used to target the fiberless radiative effector to malignant glioma cells. Once the fiberless radiative effectors have bound the tumor cell, the toxic agent acts on the cell. In some embodiments, fiberless radiative effectors containing photodynamic compounds are illuminated, resulting in the production of singlet oxygen and free radicals that diffuse out of the fiberless radiative effector to act on the biological target. In other embodiments, fiberless radiative effectors are used in concert. For example, a first biotinylated fiberless radiative effector containing a radiodynamic compound and a photodynamic compound is attached to a biological target via a molecular recognition element. A second fiberless radiative effector that is conjugated to avidin and contains a gamma-ray emitter is then attached to the first fiberless radiative effector via avidin-biotin binding. The gamma-rays emitted by the second fiberless radiative effector cause production of singlet oxygen and free radicals by the radiodynamic and photodynamic compounds in the first fiberless radiative effector. These toxic molecules then act to destroy or inhibit the growth of the biological target. Fiberless radiative effectors targeted to pathogens are used to destroy the pathogens in a similar manner.

The fiberless radiative effector is also be utilized for imaging tumors or other biological targets. In some embodiments, the fiberless radiative effectors contain a detectable material (*i.e*., luciferase or magnetic particles) that can be detected by a suitable detection system (*e.g*., bioluminescent imaging with a CCD or Magnetic Resonance Imaging). In some embodiments the magnetic fiberless radiative effectors also contain a toxic agent, so that the same fiberless radiative effector is used for therapy as well as monitoring the growth of the tumor.

It is contemplated that the use of the fiberless radiative effectors of the present invention in photodynamic tumor therapy regimes has many advantages over traditional photodynamic therapy. First, there are two critical mass aspects. The large concentration of photoactive centers in the core of the fiberless radiative effector creates a concerted photodynamic effect that destroys the cell (*e.g.,* by "punching" large holes in the membrane to which it is attached or by denaturing receptors/transporters and recognition adhesion molecules). In contrast, the isolated photodynamic molecule has to first penetrate the cell membrane and then enter a certain organelle before it can cause significant damage. Furthermore, as in chemotherapy, isolated photoactive molecules tend to leak out of the cell, be sequestered in the wrong locations, or be pumped back out of cells that express multiple drug resistance proteins. This problem is obviated by the fiberless radiative effector.

In addition, fiberless radiative effectors have several other advantages for tumor therapy. First, no aggregation of the photodynamic molecules occurs so there is no self-quenching of the excitation and no loss in singlet oxygen formation. Second, adding molecular targeting to a photodynamic molecule is quite expensive and often impractical due to interference between the two main functions - targeting and photodynamic activity. The fiberless radiative effectors of the present invention circumvent these problems and deliver many more killer radicals per target. Third, the side effects from photodynamic molecules are less of an issue when the molecule is *permanently* encapsulated inside the fiberless radiative effector. Fourth, targeting the fiberless radiative effector is orders of magnitude more efficient (i.e., the binding to the antigen has an equilibrium distribution constant that is orders of magnitude larger compared to isolated, targeted photodynamic molecules). Fifth, unlike traditional photodynamic therapy, magnetic signaling/targeting and magnetocatalysis via magnetic nanoparticles is possible with the fiberless radiative effectors of the present invention. Sixth, non-traditional methods of photodynamic therapy can be utilized with the fiberless radiative effectors of the present invention. For instance, x-rays or gamma-rays can be used for excitation, instead of standard light sources (*e.g.,* lasers). This is demonstrated with x-ray transducing materials (*e.g*., polystyrene based nanoscintillators), that transfer the energy to the photoactive molecule (one absorbed x-ray photon gives the equivalent of 500 absorbed visible photons). The very same principle works for gamma-rays. Even the visible photon absorption and quantum efficiency can be much improved by supra- or super-molecular antenna systems, in analogy to the photosynthetic antenna in green plants. The fiberless radiative effector core thus serves as an efficient amplification system. This makes it possible to use significantly reduced doses of irradiation, thus minimizing side effects. Additionally, there is practically no limit on treatable tumor depth with the use of x-rays or gamma rays for photodynamic excitation.

Furthermore, the use of photodynamic therapy for treatment of pathogen exposure is facilitated by the use of fiberless radiative effectors of the present invention. Specifically, it is contemplated that the therapeutic index of photodynamic compounds for this use will be increased by several orders of magnitude. It is contemplated that the selectivity (*i.e*., specificity) of fiberless radiative effectors results in attachment predominately to invading bacteria, which generally take up less than 10⁻⁸ of the body's weight (based on less than 10⁴ bacteria/ml blood). Therefore, one needs only about one in 10⁸ of the photodynamic compound dose usually administered (intravenously). To be conservative, this fraction may be increased to about 10⁻⁶, and in order to reduce by a factor of 1000 the needed irradiation time *(i.e.,* from about 10⁻³ to 1 second), it can be increased further to 10⁻³ (*i.e.,* 0.1 mg total dye compared to traditional doses of 100 mg or higher). It is contemplated that this eliminates undesired side effects. In contrast to individual photodynamic compounds, in some embodiments fiberless radiative effectors do not enter cells, except possibly macrophage cells (*i.e*., when the "cloaking" fails). At the projected short irradiation times, one single macrophage will have to incorporate thousands of fiberless radiative effectors to be photodamaged. Even if the majority of the fiberless radiative effectors are taken up by macrophages, the integrated damage to the macrophage population will be negligible. As long as one ppm of the fiberless radiative effectors escape the macrophages and find their target, most bacteria will be photoinactivated.

Methods for preparing basic fiberless radiative effectors are described below, followed by descriptions of toxic agents, cloaking materials, and molecular recognition elements that may be utilized with the fiberless radiative effectors. Uses of the fiberless radiative effectors are then discussed in detail.

### I. Matrix Materials for Fiberless Radiative Effectors

The fiberless radiative effectors of the present invention are preferably small particles (*i.e.*, fiberless) with a core matrix. The present invention contemplates the fabrication of the fiberless radiative effector from matrix materials in which toxic agents are incorporated or embedded, to which molecular recognition elements are attached, and that are cloaked or placed in a cloaking material. It is also desirable that the matrix material allow the diffusion of free radicals and other small molecules into and out of the particle the fiberless radiative effector. The matrix material of the present invention is also biologically compatible (i.e., non-toxic to biological systems) and should allows energy transduction (i.e., allow the passage of photons through the material).

A variety of matrix materials are contemplated. In some embodiments, a polymeric matrix (*e.g.,* polyacrylamide or poly(vinyl chloride)) is utilized, while in other embodiments a glass matrix is utilized (*e.g*., sol-gel). In some embodiments of the present invention, the fiberless radiative effectors are finely ground or formed particles, with sizes ranging from 1 nm to 5 µm, preferably from about 40 to 400 nm. The matrices can be hydrophobic (*e.g*., decyl-methacrylate), hydrophilic (*e.g.*, polyacrylamide), or biodegradable (*e.g*., poly(lactic acid)).

In some embodiments, fiberless radiative effectors are fabricated from polyacrylamide. In some preferred embodiments, about 27% acrylamide and about 3% N,N-methylenebis(acrylamide) are combined in a buffer (*e.g*., 0.1M phosphate buffer, pH 6.5). About one ml of this solution is then added to a solution containing about 20 ml hexane, 1.8 mM sodium dioctyl sulfosuccinate, and about 4.24 mM Brij 30 *(i.e.,* 4 lauryl ether). The solution is then stirred under nitrogen for 20 minutes while cooling in an ice bath. The polymerization is initiated with about 24 µl of a 10% ammonium persulfate solution and about 12 µl N.N,N',N'-tetraethyldiethylenetriamine (TEMED). The solution is then stirred at room temperature for about 2 hours. Hexane is removed by rotary evaporation, and the probes are rinsed free of surfactant with ethanol. This procedure yields particles of about 20 to 200 nm.

In other embodiments, fiberless radiative effectors are fabricated from poly(vinyl chloride). In some preferred embodiments, the poly(vinyl chloride) (*e.g*., about 33 wt %) is combined with a plasticizer (*e.g*., about 66 wt %) and dissolved in a solvent (*e.g*., about 200 mg of poly(vinyl chloride)/plasticizer mixture is added to 5 ml freshly distilled tetrahydrofuran (THF)). The solution is then coated onto polystyrene spheres of the desired size (*e.g*., about 10-1000 nm), and the coated spheres ground in liquid nitrogen.

In still other embodiments, the fiberless radiative effectors are fabricated from decymethacrylate. In some preferred embodiments, a polymerization solution is made by combining about 55 mg of decyl-methacrylate and about 75 mg of hexanediol dimethacrylate. The solution is then washed three times with 5% sodium hydroxide solution and three times with water. About 100 mg of dioctyl sebacate (DOS), 5 mg of benzophenone, and 2.5 mg of benzoyl peroxide are added to the washed monomer. This solution is then added to 1 ml of water and sonicated for 1 hour until uniform. The spheres are then purged with nitrogen for 20 min, and polymerized with a UV lamp for 15 min.

In still other embodiments, the fiberless radiative effectors are fabricated using a sol-gel procedure. In some preferred embodiments, a polymerization solution is prepared comprising about 1.5 g sodium bis(2-ethylhexyl) sulfosuccinate (AOT), about 3 g Brij 30, about 9 ml hexane, about 4.5 ml tetraethylorthosilicate (TEOS), and about 250 ml NH₃H₂O. The solution is allowed to react for 18 hrs, with stirring, at room temperature.

In some preferred embodiments, a co-polymer with functionalized groups or otherwise reactive groups (*e.g*., amine groups) is included during the fabrication of the fiberless radiative effector. The co-polymer allows the convenient attachment of molecular recognition elements and other compounds (*e.g*., cloaking materials, biotin) to the fiberless radiative effector. Useful co-polymers include (N-(3-Aminopropyl)methacrylamide hydrochloride) (APMA), acryloamidodextran, aminomethylstyrene, 2-hydroxyethyl acrylate, 2-hydroxymethacrylate, 4-hydroxybutyl acrylate (*See e.g.,* Daubresse *et al.,* J. Pharm. Pharmacol. 45(12):1018-23 [1993]; Noguchi *et al.,* J. Biomed. Mat. Res. 39(4):621-9 [1998]).

### II. Toxic Agents for Incorporation Into Fiberless Radiative Effectors

A variety of toxic agents are incorporated into the fiberless radiative effector. The toxic agents may themselves be toxic, or they may act in concert with another compound, material, or external stimulus to produce a toxic compound, material, or molecule. The toxic agents are preferably incorporated into the fiberless radiative effectors by inclusion in the polymerization mixtures described above. In some embodiments, the toxic agents include photodynamic or radiodynamic compounds. Some preferred photodynamic compounds include those that can participate in a type II photochemical reaction:

PS + hν → PS*(1)

PS*(1) → PS*(3)

PS*(3) + O₂ → PS + *O₂

*O₂ + T → cytotoxity

where PS = photosenstizer, PS*(1) = excited singlet state of PS, PS*(3) = excited triplet state of PS, hv = light quantum, *O₂ = excited singlet state of oxygen, and T = biological target. Other photodynamic compounds useful in the present invention include those that cause cytotoxity by a different mechanism than singlet oxygen production (*e.g*., copper benzochlorin, Selman, *et al.,* Photochem. Photobiol., 57:681-85 [1993]). Examples of photodynamic compounds that find use in the present invention include Photofrin 2, phtalocyanins *(See e.g.,* Brasseur *et al.,* Photochem. Photobiol., 47:705-11 [1988]), benzoporphyrin, tetrahydroxyphenylporphyrins, naphtalocyanines *(See e.g.,* Firey and Rodgers, Photochem. Photobiol., 45:535-38 [1987]), sapphyrins (Sessler *et al.,* Proc. SPIE, 1426:318-29 [1991]), porphinones (Chang *et al.,* Proc. SPIE, 1203:281-86 [1990]), tin etiopurpurin, ether substituted porphyrins (Pandey *et. al.,* Photochem. Photobiol., 53:65-72 [1991]), and cationic dyes such as the phenoxazines (*See e.g.,* Cincotta *et al.,* SPIE Proc., 1203:202-10 [1990]).

In other embodiments, toxic agents that directly produce free radicals (*i.e*., do not produce singlet oxygen) are incorporated into the fiberless radiative effectors during polymerization. This approach allows for larger and longer lived fiberless radiative effectors and will not be limited by local oxygen supplies. Such toxic agents include 2-methyl-4-nitro-quinoline-N-oxide (Aldrich) and 4,4-dinitro-(2,2) bipyridinyl- N,N dioxide (Aldrich), which produce hydroxyl radicals when illuminated with 360-400 nm light (Botchway *et al.,* Photochem. Photobiol. 67(7):635-40 [1998]); malachite green and isofuran blue (Molecular Probes), which produce hydroxyl radicals upon stimulation with about 630 nm light (Jay *et al.,* PNAS 91:2659 [1994]; Haugland, Handbook of Fluorescent Probes and Research Chemicals, 6th ed., Molecular Probes, Eugene, OR [1994]); potassium nitrosylpentachlororuthenate (Molecular Probes) (abs = 516 nm), Roussin's black salt and Roussin's red salt (abs 313-546 nm), serve as sources of NO which is toxic to cells (Murphy *et al.,* Neuropharm. 33:1375-85 [1994]; Bourassa *et al.,* JACS 119:2853-60 [1997]); and other photolytic nitric oxide and hydroxyl donors (De Leo and Ford, JACS 121:1980-81 [1999]).

In still other embodiments, the toxic agents are preferably radiodynamic compounds. Fiberless radiative effectors incorporating radiodynamic compounds preferably comprise a polystyrene or polystyrene like matrix *(e.g*., poly(styrene)-poly(vinylpyridine) that serves as a molecular antennae *(See e.g.,* Kokotov *et al.,* Photochem. Photobiol. 59(3):385-87 [1994] ). Additionally, the radiodynamic fiberless radiative effectors preferably include a sensitizer such as a scintillating compound (*e.g*., NaI-125, 2,5-diphenyloxazole (PPO); 2-(4-biphenyl)-6-phenylbenzoxazole; 2,5-bis-(5'-tert-butylbenzoxazoyl-[2'])thiophene; 2-(4-t-butylphenyl)-5-(4-biphenylyl)-1,3,4-oxadiazole; 1,6-diphenyl-1,3,5-hexatriene; trans-p,p'-diphenylstilbene; 2-(1-naphthyl)-5-phenyloxazole; 2-phenyl-5-(4-biphenylyl)-1,3,4-oxadiazole; p-terphenyl; and 1,1,4,4-tetraphenyl-1,3-butadiene) and a porphyrin containing compound (*e.g*., hematoporphyrin or Photofrin II). It is believed that the molecular antennae is excited by x-ray or gamma ray photons to produce excitons. The excitons are then transferred to the sensitizer and then to the porphyrin or other photodynamic compound. This transfer of energy results in the production of singlet oxygen and other free radicals as described above. It is believed that the fiberless radiative effectors of the present invention cause cell death or apoptosis due to the generation of singlet oxygen and other free radicals (*e.g.,* superoxide and hydroxyl radical) that diffuse from the fiberless radiative effector to interact with the biological target. With respect to tumors, it is thought that the tumors are initially sublethally damaged, and then die when subsequent vascular collapse results in tumor hypoxia.

### III. Other Materials for Inclusion in Fiberless Radiative Effectors

In some embodiments, it is contemplated that the fiberless radiative effector further comprise a radioactive element or compound that emits gamma rays (*e.g.,* ¹¹¹In-oxine, ⁵⁹Fe, ⁶⁷Cu, ¹²⁵I, ⁹⁹Te (Technetium), and ⁵¹Cr) or elements or compounds that emit beta particles (*e.g.,* ³²p, ³H, ³⁵S, ¹⁴C, ⁹⁰Y). Fiberless radiative effectors containing such elements are utilized as a discreet energy source for the excitation of a separate fiberless radiative effector that includes a radiodynamic or photodynamic compound. In some embodiments, the fiberless radiative effector also includes a scintillating material so that photons are produced as a result of the emission of the beta particles or gamma rays. Some of these photons are transmitted to the fiberless radiative effector containing a photodynamic dye so that singlet oxygen and other radicals are produced.

In other embodiments of the present invention, the radioactive element or compound is preferably the toxic agent. In some embodiments, fiberless radiative effectors containing a radioactive element are used for internal radiation therapy *(See e.g.,* Yan *et al.,* Cancer 72(11):3210-15 [1993]; Ariel *et al.,* J. Surg. Oncol. 20:151-6 [1982]; Ehrhardt and Day, Nucl. Med. Biol. 14(3):233-42 [1987]).

In other embodiments of the present invention, the fiberless radiative effector preferably includes dendrimeric molecules (*See e.g.,* Swallen *et al.,* J. Luminescence 76&77:193-96 [1998]; Bar-Haim *et al.,* JACS 119:6197-98 [1997]). Dendrimeric molecules are composed of repeating units (*e.g.,* phenylacetylene), growing exponentially in successively larger generations out from a central core. Dendrimers are specified by three main characteristics: the basic chemical building elements (*e.g*., phenylacetylene); the branching (*i.e.,* coordination) number, Z, at each node, and the number of generations. Each successive generation contains a factor of (Z-1) more elements than the previous one, and thus the total number of elements increases exponentially with generation number. It is believed that the dendrimer molecules act as antennas that funnel absorbed light to the photodynamic compound. In some embodiments of the present invention, the incorporation of dendrimer molecules into the fiberless radiative effectors increases photostability.

In some embodiments of the present invention, the fiberless radiative effectors preferably includes a protein (*e.g*., luciferase) that catalyzes a reaction that produces a photon (i.e., bioluminescence). Many different luciferases are incorporated into the fiberless radiative effectors of the present invention, including bacterial luciferase (U.S. Pat. No. 4,548,994), *Photinus pyralis* luciferase (U.S. Pat. Nos. 5,670,356 and 5,674,713), *Renilla reniformus* luciferase, *Pyrophorus plagiophthalamus* luciferase, *Luciola cruciata* luciferase (Masuda *et al.,* Gene 77:265-70 [1989]), *Luciola lateralis* luciferase (Tatsumi *et al.,* Biochim. Biophys. Acta 1131:161-65 [1992]), and *Latia neritoides* luciferase. In some preferred embodiments, luciferase is incorporated into sol-gel or polyacrylamide fiberless radiative effectors. The luciferase-containing fiberless radiative effectors find use in the present invention for labelling biological targets so that the target is visualized following excitation with light of the appropriate wavelength in the presence of appropriate substrates (*e.g.,* luciferin and ATP).

In other embodiments of the present invention, the fiberless radiative effectors preferably include an antioxidant or antifade agent. Antioxidants are preferably only included in fiberless radiative effectors not containing a photodynamic or radiodynamic compound, as the antioxidant would quench singlet oxygen and free radical production. However, the inclusion of antioxidants in fiberless radiative effectors containing, for instance, radioactive elements, is desirable so that the fiberless radiative effector is protected from oxidation. Suitable antioxidants or antifade agents include ascorbic acid, vitamin C, E, beta-carotene and their derivitives and other dietary antioxidants, phenylalanine, azide, p-phenylenediamine, n-propylgallate, diazabicyclo[2,2,2]octane, and the commercial reagents SlowFade and ProLong (Molecular Probes, Eugene OR).

### IV. Cloaking Materials for Fiberless Radiative Effectors

A variety of cloaking materials or agents find use with the fiberless radiative effectors of the present invention, including, poly(ethylene glycol), liposomes, poly(ethylene glycol) coated liposomes, oxidized dextran, and red blood cell ghosts. Preferably, the cloaking material protects the fiberless radiative effector from recognition by the immune system and circulatory reticulo-endothelial system (RES). This results in both increased delivery of the fiberless radiative effector to the intended biological target and reduced toxicity to other body tissues.

Many suitable poly(ethylene glycol)(PEG) based materials are known *(See e.g.,* Zalipsky, Bioconjug. Chem. 6:150-65 [1995]; Coombes *et al.,* Biomaterials 18:1153-61 [1997]; Vandorpe *et al.,* Biomaterials 18:1147-52 [1997]; and Zalipsky *et al.,* J. Cont. Release 39:152-61 [1996]). The present invention contemplates such PEG-based materials as cloaking agents. In some preferred embodiments, derivatized or functionalized PEG (*e.g.,* amino-PEG, sulfonate esters of PEG, hydrazido-PEG, and carboxyl-PEG) is utilized to cloak the fiberless radiative effector. Other cloaking materials include poloxamer and poloxamine block co-polymers (*See e.g.,* Torchilin, J. Microencapsulation 15(1) 1-19 [1998]) such as poly(phosphazene)-poly(ethylene oxide)(Vandorpe *et al., supra*), and poly(ethylene oxide)-poly(propylene oxide)(Coombes *et al., supra*).

In some embodiments, the functionalized PEG or other cloaking material is covalently bound to the polymer or copolymer (*e.g.,* APMA) forming the fiberless radiative effector by utilizing the appropriate homobifunctional (*e.g.,* disuccinimidyl suberate, bis(sulfosuccinymidyl)suberate, disuccinimydal glutarate, dimethyladipimidate-2HCl, or dimethylpimelimidate-2HCl) or heterobifunctional reagent (*e.g.,* succinimydyl 3-(2-pyridyldithio)propionate (SPDP); succinimidyl trans-4-(N-maleimidylmethyl)cyclohexane-1-carboxylate (SMCC); succinimidyl (acetylthio)acetate (SATA); 4-[(succinimydyloxy)carboxyl]-α-methyl-α-(2-pyridyldithio)toluene (SMPT); succinimidyl 4-[[(iodoacetyl0amino]methyl]-cyclohexane-1-carboxylate (SIAC); m-maleimidobenzoyl-N-hydroxysuccinimide ester (MBS), succinimydyl 4-(p-maleimdophenyl)-butyrate (SMPB) and succinimidyl *p*-azidobenzoate (SAB)). In other embodiments, the fiberless radiative effectors are first silanized (*e.g.,* with 3-(trimethoxysilyl)propyl methacrylate, Barker *et al.,* Anal. Chem. 70:971-76 [1998]; Barker *et al.,* Anal. Chem. 69:990-95 [1997]. Functionalized PEG or other cloaking materials can then be covalently bound to the silanized fiberless radiative effector.

In still other embodiments, the cloaking material is a liposome. In some preferred embodiments, a plurality of fiberless radiative effectors are contained within the liposome. A variety of methods are known for producing liposomes with good stability in the circulatory system. In some embodiments, liposomes are sterically stabilized by incorporation of the end group functionalized PEG-lipid derivative pyridylthiopropionoylamino-PEG-distearoylphosphatidylethanolamine (PDP-PEG-DSPE)(*See e.g*., Allen *et al.,* Biochim. Biophys. Acta 1237:99-108 [1995]. In other embodiments, liposomes are fabricated that are sterically stabilized with disteroyl-N-(3-carboxypropionoyl poly(ethylene glycol) succinyl)phosphatidylethanolamine (DSPE-PEG-COOH)(*See e.g.,* Maruyama *et al.,* Biochim. Biophys. Acta 1234:74-80 [1995].

In some embodiments, molecular recognition elements are covalently attached to the stabilized liposome.

In other embodiments, the cloaking material is a red blood cell ghost. In some preferred embodiments, a plurality of fiberless radiative effectors are contained within the red blood cell ghosts *(See e.g.,* Matteo *et al.,* Am. J. Obstet. Gynecol. 178(2):402-08 [1998]; Heinz and Hoffman, J. Cell Comp. Physiol. 65:31-44 [1965]). An advantage of red blood cell ghosts is that integral membrane proteins remain in place and can be used as a convenient means for covalent attachment of molecular recognition elements.

### V. Molecular Recognition Elements for Fiberless Radiative Effectors

In other embodiments of the present invention, a molecular recognition element is attached, fixed or conjugated to the fiberless radiative effector so that it can be targeted to a particular biological target. It is contemplated that the fiberless radiative effectors of the present invention are targeted via the molecular recognition element to a variety of biological targets, including tumor cells, bacteria, viruses, cell surface proteins, cell surface receptors, cell surface polysaccharides, extracellular matrix proteins, intracellular proteins and intracellular nucleic acids.

Examples of molecular recognition elements that find use in the present invention include nucleic acids (*e.g.,* RNA and DNA), polypeptides (*e.g*., receptor ligands, signal peptides, avidin, Protein A, antigen binding proteins), polysaccharides, biotin, hydrophobic groups, hydrophilic groups, drugs and any organic molecules that bind to receptors. It is contemplated that the fiberless radiative effectors of the present invention display (*i.e.,* be conjugated to) one, two, or a variety of molecular recognition elements.

Utilization of more than one molecular recognition element in a fiberless radiative effector allows multiple biological targets to be targeted. Multiple molecular recognition elements also allows the fiberless radiative effectors to be utilized in a "stacking" approach wherein a first fiberless radiative effector (*e.g.,* comprising a radiodynamic compound) is targeted to a biological target, and a second fiberless radiative effector (*e.g*., comprising a gamma-ray emitter and avidin as a molecular recognition element) is targeted to a second molecular recognition element (*e.g*., biotin) on the first fiberless radiative effector.

### A. Peptides that Specifically Target Tumor Cells

In some preferred embodiments, the fiberless radiative effector molecular recognition elements comprise peptides that bind specifically to tumor blood vessels *(See e.g.,* Arap *et al.,* Science 279:377-80 [1998]). These peptides include, but are not limited to, peptides containing the RGD (Arg-Gly-Asp) motif (*e.g.,* CDCRGDCFC; SEQ ID NO:1), the NGR (Asn-Gly-Arg) motif (*e.g*., CNGRCVSGCAGRC; SEQ ID NO:2), or the GSL (Gly-Ser-Leu; SEQ ID NO:3) motif. These peptides and conjugates containing these peptides selectively bind to various tumors, including breast carcinomas, Karposi's sarcoma, and melanoma. It is believed that these peptides are ligands for integrins and growth factor receptors that are absent or barely detectable in established blood vessels. In some preferred embodiments, the peptide is preferably produced using chemical synthesis methods. For example, peptides can be synthesized by solid phase techniques, cleaved from the resin, and purified by preparative high performance liquid chromatography *(See e.g.,* Creighton (1983) Proteins Structures And Molecular Principles, W H Freeman and Co, New York N.Y.). In other embodiments of the present invention, the composition of the synthetic peptides is confirmed by amino acid analysis or sequencing *(See e.g*., Creighton, *supra).*

### B. Peptides that Specifically Target Gliomas

In some preferred embodiments, the fiberless radiative effector molecular recognition elements comprise peptides that specifically bind to glioma cells (*See e.g.,* Debinski *et al.,* Nature Biotech. 16:449-53 [1998]; Debinski *et al.,* J. Biol. Chem. 270(28):16775-80 [1995]; and Debinski *et al.,* J. Biol. Chem. 271(37):22428-33 [1996]). In some embodiments, the present invention contemplates fiberless radiative effectors comprising IL13 or one of its variants so that the fiberless radiative effector will bind to IL13 binding sites in glioma cells.

Human high-grade gliomas are uniquely enriched in IL13 binding sites. Many of the established brain tumor cell lines, primarily malignant gliomas, over-express hIL13 binding sites. Human malignant glioma cell lines express high number, up to 30,000, binding sites for hIL13 per cell. Of interest, glioblastoma multiforme (GBM) explant cells showed an extraordinary high number of hIL13 binding sites, up to 500,000 per cell. The binding of hIL13 is not neutralized by hIL4 on an array of established human glioma cell lines that includes U-251 MG, U-373 MG, DBTRG MG, Hs-683, U-87 MG, SNB-19, and A-172 cells.

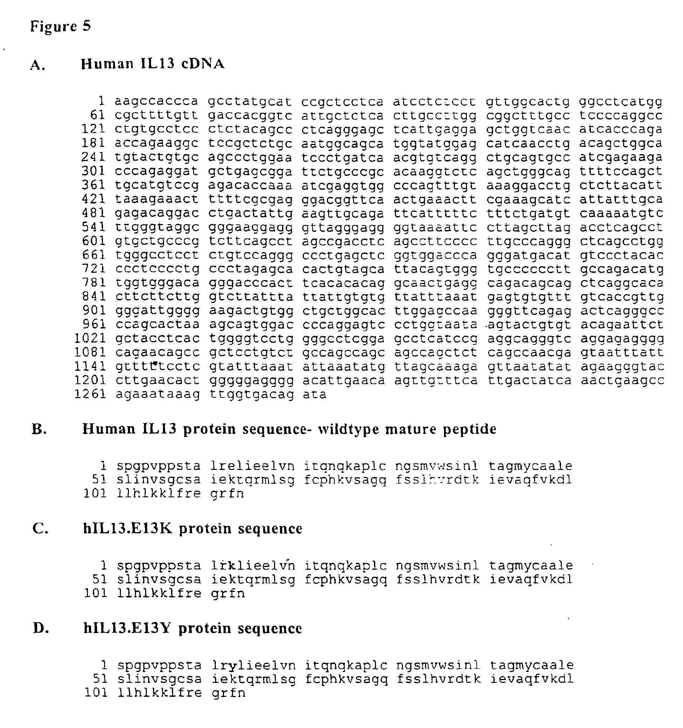

hIL13 (SEQ ID NO:4, *see* Fig. 7) can be engineered to increase its specific targeting of high-grade gliomas. The pattern for IL13- and IL4R sharing on normal cells requires IL13 to bind hIL4R This is confirmed by the fact that hIL13 binding is always fully competed by hIL4. The recently proposed model for this hIL13R suggests that the shared hIL13/4R is heterodimeric. This scenario would imply that hIL13 may contain at least two receptor-binding sites, each recognizing a respective subunit of the receptor. The engineered hIL13 variants (*e.g.,* hIL13.E13K (SEQ ID NO:5) or hIL13.E13Y (SEQ ID NO:6), *see* Fig. 5) are deprived of cell signaling abilities (Debinski *et al.,* [1998], *supra*). This is desirable because interaction with physiological systems contributes prominently to the dose-limiting toxicity of some biological therapeutics (*e.g*., cytokines). Significantly, the molecule of hIL13 appears not to be sensitive to a variety of genetically engineered modifications and these variants can be produced in large quantities. It is thus possible to divert the molecule of hIL13 from its physiological receptor and make it a non-signaling compound, while its affinity toward the HGG-associated receptor remains intact or is increased. Such forms of IL13 can serve as rationally designed vectors for variety of imaging and therapeutic approaches of HGG.

Given the grim prognosis following the identification of an intracranial malignancy, any strategy for the pre-, intra- or post-operative identification and removal of cancer cells is a significant improvement. Recent discovery of the expression of IL-13 receptors on the surface of all of the malignancies of glial origin provides a novel strategy for the accumulation and retention of fiberless radiative effectors within CNS cancers. The high-grade glioma-associated receptor for IL13 used in the present invention is much more specific than the receptor for either transferrin or EGF, since its normal tissue presence is negligible or null. Also, the over-expression of the IL13R in gliomas is much more frequent and homogenous than that for EGF.

In some embodiments, nucleic acids (*e.g.,* SEQ ID NO:7) encoding IL13 fragments, fusion proteins or functional equivalents or variants (*e.g.,* hIL13.E13K or hIL13.E13Y) thereof are cloned into an appropriate expression vector, expressed and purified (*e.g*., preferably as described in Debinski *et al.,* Nature Biotech. 16:449-53 [1998]; Debinski *et al.,* J. Biol. Chem. 270(28):16775-80 [1995]; and *Debinski et al.,* J. Biol. Chem. 271(37):22428-33 [1996]). In other embodiments of the present invention, vectors include chromosomal, nonchromosomal and synthetic DNA sequences, *e.g*., derivatives of SV40; bacterial plasmids; phage DNA; baculovirus; yeast plasmids; vectors derived from combinations of plasmids and phage DNA, viral DNA such as vaccinia, adenovirus, fowl pox virus, and pseudorabies. Large numbers of suitable vectors are known to those of skill in the art, and are commercially available. Such vectors include the following vectors: 1) Bacterial - pQE70, pQE60, pQE-9 (Qiagen), pBS, pD10, phagescript, psiX174, pbluescript SK, pBSKS, pNH8A, pNH16a, pNH18A, pNH46A (Stratagene); ptrc99a, pKK223-3, pKK233-3, pDR540, pRIT5 (Pharmacia); and 2) Eukaryotic - pWLNEO, pSV2CAT, pOG44, PXT1, pSG (Stratagene) pSVK3, pBPV, pMSG, pSVL (Pharmacia). Any other plasmid or vector can be used as long as they are replicable and viable in the host. In some preferred embodiments of the present invention, mammalian expression vectors comprise an origin of replication, a suitable promoter and enhancer, and any necessary ribosome binding sites, polyadenylation site, splice donor and acceptor sites, transcriptional termination sequences, and 5' flanking nontranscribed sequences. In other embodiments, DNA sequences derived from the SV40 splice, and polyadenylation sites are used to provide the required nontranscribed genetic elements.

In other embodiments, the IL13 peptide or variant thereof is expressed in a host cell. In some embodiments of the present invention, the host cell is a higher eukaryotic cell (*e.g*., a mammalian or insect cell). In other embodiments of the present invention, the host cell is a lower eukaryotic cell (*e.g*., a yeast cell). In still other embodiments of the present invention, the host cell can be a prokaryotic cell (*e.g.,* a bacterial cell). Specific examples of host cells include, *Escherrichia coli, Salmonella typhimurium, Bacillus subtilis,* and various species within the genera *Pseudomonas, Streptomyces,* and *Staphylococcus,* as well as, *Saccharomycees cerivisiae, Schizosaccharomycees pombe, Drosophila* S2 cells, *Spodoptera* Sf9 cells, Chinese Hamster Ovary (CHO) cells, COS-7 lines of monkey kidney fibroblasts, (Gluzman, Cell, 23:175 [1981]), C127, 3T3, HeLa and BHK cell lines.

In some embodiments of the present invention, IL13 or variants thereof are recovered or purified from recombinant cell cultures by methods including, but not limited to, ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. In other embodiments of the present invention, protein refolding steps are used, as necessary, in completing configuration of the mature protein. In still other embodiments of the present invention, high performance liquid chromatography (HPLC) is employed for final purification steps.

Some embodiments of the present invention provide polynucleotides having the coding sequence fused in frame to a marker sequence that allows for purification of the polypeptide of the present invention. An example of a marker sequence is a hexahistidine tag that is supplied by a vector, preferably a pQE-9 vector, that provides for purification of the polypeptide fused to the marker in the case of a bacterial host, or, for example, the marker sequence may be a hemagglutinin (HA) tag when a mammalian host (*e.g*., COS-7 cells) is used. The HA tag corresponds to an epitope derived from the influenza hemagglutinin protein (Wilson, *et al.,* Cell, 37:767 [1984]).

### D. Signal Peptides As Molecular Recognition Elements

In some embodiments of the present invention, the molecular recognition element is preferably a signal peptide. These peptides are chemically synthesized or cloned, expressed and purified as described above. The signal peptides are used to target the fiberless radiative effector to a discreet region within a cell. In some embodiments, fiberless radiative effectors with signal peptides as molecular recognition elements are delivered to cells via targeted liposomes.

Specific amino acid sequences in proteins are responsible for targeting the polypeptide into cellular organelles and compartments. In some embodiments, the signal peptides direct protein import into mitochondria. In further embodiments, the molecular recognition element is preferably: NH- Met-Leu-Ser-Leu-Arg-Gln-Ser-Ile-Arg-Phe-Phe-Lys-Pro-Ala-Thr-Arg-Thr-Leu-COOH (SEQ ID NO:8).

It is contemplated that this peptide forms an amphipathic helix that associates with mitochondrial membranes sites of protein import. This allows the peptide-fiberless radiative complex to attach to mitochondrial membranes. It is unlikely that the complex will be internalized, since there are few pores of nm size on intact mitochondria.

In still other embodiments, the following nuclear localization signal is utilized: NH-Pro-Pro-Lys-Lys-Lys-Arg-Lys-Val-COOH (SEQ ID NO:9). In another embodiment, SNAP-25, is utilized to deliver fiberless radiative effectors to the presynaptic region of neuronal cells. It is contemplated that SNAP-25 is one of the prototypic v-SNARE proteins. SNAP-25 localizes specifically to the presynaptic terminals of neuronal cells and PC-12 cells in culture. It is not known which portion of the peptide is responsible for sorting to the presynaptic terminal. However, during cellular processing of the peptide, SNAP-25 becomes palmitoylated at a central Cys-quartet. These palmitylated groups help anchor the protein in the presynpatic membrane. SNAP-25 associates with syntaxin, and ultimately, with the entire vesicular fusion machinery in a calcium-activated presynaptic terminal.

### E. Antibodies as Molecular Recognition Elements

In some embodiments of the present invention, the molecular recognition elements are preferably antigen binding proteins or antibodies. Antibodies can be generated to allow for the targeting of antigens or immunogens (*e.g*., tumor , tissue or pathogen specific antigens) on various biological targets (*e.g.,* pathogens, tumor cells, normal tissue). Such antibodies include, but are not limited to polyclonal, monoclonal, chimeric, single chain, Fab fragments, and an Fab expression library.

In some preferred embodiments, the antibodies recognize tumor specific epitopes (e.g., TAG-72 (Kjeldsen *et al.,* Cancer Res. 48:2214-2220 [1988]; U.S. Pat. Nos. 5,892,020; 5,892,019; and 5,512,443); human carcinoma antigen (U.S. Pat. Nos. 5,693,763; 5,545,530; and 5,808,005); TP1 and TP3 antigens from osteocarcinoma cells (U.S. Pat. No. 5,855,866); Thomsen-Friedenreich (TF) antigen from adenocarcinoma cells (U.S. Pat. No. 5,110,911); "KC-4 antigen" from human prostrate adenocarcinoma (U.S. Pat. Nos. 4,708,930 and 4,743,543); a human colorectal cancer antigen (U.S. Pat. No. 4,921,789); CA125 antigen from cystadenocarcinoma (U.S. Pat. No. 4,921,790); DF3 antigen from human breast carcinoma (U.S. Pat. Nos. 4,963,484 and 5,053,489); a human breast tumor antigen (U.S. Pat. No. 4,939,240); p97 antigen of human melanoma (U.S. Pat. No. 4,918,164); carcinoma or orosomucoid-related antigen (CORA)(U.S. Pat. No. 4,914,021); a human pulmonary carcinoma antigen that reacts with human squamous cell lung carcinoma but not with human small cell lung carcinoma (U.S. Pat. No. 4,892,935); T and Tn haptens in glycoproteins of human breast carcinoma (Springer *et al.,* Carbohydr. Res. 178:271-292 [1988]), MSA breast carcinoma glycoprotein termed (Tjandra *et al.,* Br. J. Surg. 75:811-817 [1988]); MFGM breast carcinoma antigen (Ishida *et al.,* Tumor Biol. 10:12-22 [1989]); DU-PAN-2 pancreatic carcinoma antigen (Lan *et al.,* Cancer Res. 45:305-310 [1985]); CA125 ovarian carcinoma antigen (Hanisch *et al.,* Carbohydr. Res. 178:29-47 [1988]); YH206 lung carcinoma antigen (Hinoda *et al.,* (1988) Cancer J. 42:653-658 [1988]).

In other preferred embodiments, the antibodies recognize specific pathogens (*e.g., Legionella peomophilia, Mycobacterium tuberculosis, Clostridium tetani, Hemophilus influenzae, Neisseria gonorrhoeae, Treponema pallidum, Bacillus anthracis, Vibrio cholerae, Borrelia burgdorferi, Cornebacterium diphtheria, Staphylococcus aureus,* human papilloma virus, human immunodeficiency virus, rubella virus, polio virus).

Various procedures known in the art are used for the production of polyclonal antibodies. For the production of antibody, various host animals can be immunized by injection with the peptide corresponding to the desired epitope including rabbits, mice, rats, sheep, goats, etc. In a preferred embodiment, the peptide is conjugated to an immunogenic carrier (*e.g*., diphtheria toxoid, bovine serum albumin (BSA), or keyhole limpet hemocyanin (KLH)). Various adjuvants are used to increase the immunological response, depending on the host species, including Freund's (complete and incomplete), mineral gels such as aluminum hydroxide, surface active substances such as lysolecithin, pluronic polyols, polyanions, peptides,oil emulsions, keyhole limpet hemocyanins, dinitrophenol, and potentially useful human adjuvants such as BCG (Bacille Calmette-Guerin) and *Corynebacterium parvum.*

For preparation of monoclonal antibodies, any technique that provides for the production of antibody molecules by continuous cell lines in culture may be used *(See, e.g.,* Harlow and Lane, *Antibodies: A Laboratory Manual, Cold Spring Harbor Laboratory Press,* Cold Spring Harbor, NY). These include the hybridoma technique originally developed by Köhler and Milstein (Köhler and Milstein, Nature 256:495-497 [1975]), as well as the trioma technique, the human B-cell hybridoma technique *(See e.g.,* Kozbor *et al.* Immunol. Today 4:72 [1983]), and the EBV-hybridoma technique to produce human monoclonal antibodies (Cole *et al., in Monoclonal Antibodies and Cancer Therapy,* Alan R Liss, Inc., pp. 77-96 [1985]).

In an additional embodiment of the invention, monoclonal antibodies can be produced in germ-free animals utilizing recent technology *(See e.g.,* PCT/US90/02545). According to the invention, human antibodies may be used and can be obtained by using human hybridomas (Cote *et al.,* Proc. Natl. Acad. Sci. U.S.A.80:2026-2030 [1983]) or by transforming human B cells with EBV virus *in vitro* (Cole *et al., in Monoclonal Antibodies and Cancer Therapy,* Alan R. Liss, pp. 77-96 [1985]).

According to the invention, techniques described for the production of single chain antibodies (U.S. Patent 4,946,778) can be adapted to produce specific single chain antibodies. An additional embodiment of the invention utilizes the techniques described for the construction of Fab expression libraries (Huse *et al.,* Science 246:1275-1281 [1989]) to allow rapid and easy identification of monoclonal Fab fragments with the desired specificity.

Antibody fragments that contain the idiotype (antigen binding region) of the antibody molecule can be generated by known techniques. For example, such fragments include the F(ab')2 fragment that can be produced by pepsin digestion of the antibody molecule; the Fab' fragments that can be generated by reducing the disulfide bridges of the F(ab')2 fragment, and the Fab fragments that can be generated by treating the antibody molecule with papain and a reducing agent.

In the production of antibodies, screening for the desired antibody can be accomplished by techniques known in the art (*e.g*., radioimmunoassay, ELISA (enzyme-linked immunosorbant assay), "sandwich" immunoassays, immunoradiometric assays, gel diffusion precipitin reactions, immunodiffusion assays, *in situ* immunoassays (using colloidal gold, enzyme or radioisotope labels, for example), Western Blots, precipitation reactions, agglutination assays (*e.g*., gel agglutination assays, hemagglutination assays, etc.), complement fixation assays, immunofluorescence assays, protein A assays, and immunoelectrophoresis assays).

### F. Other Molecular Recognition Elements

The molecular recognition elements of the fiberless radiative effectors of the present invention may recognize a variety of other epitopes on biological targets (*e.g.,* pathogens, tumor cells, normal tissues). In some embodiments, molecular recognition elements are incorporated to recognize, target or detect a variety of pathogenic organisms including, sialic acid to target HIV (Wies *et al.,* Nature 333: 426 [1988]), influenza (White *et al.,* Cell 56: 725 [1989]), *Chlamydia* (Infect. Imm. 57: 2378 [1989]), *Neisseria meningitidis, Streptococcus suis, Salmonella,* mumps, newcastle, and various viruses, including reovirus, Sendai virus, and myxovirus; and 9-OAC sialic acid to target coronavirus, encephalomyelitis virus, and rotavirus; non-sialic acid glycoproteins to detect cytomegalovirus (Virology 176: 337 [1990]) and measles virus (Virology 172: 386 [1989]); CD4 (Khatzman *et al.,* Nature 312: 763 [1985]), vasoactive intestinal peptide (Sacerdote *et al.,* J. of Neuroscience Research 18: 102 [1987]), and peptide T (Ruff *et al.,* FEBS Letters 211: 17 [1987]) to target HIV; epidermal growth factor to target vaccinia (Epstein *et al.,* Nature 318: 663 [1985]); acetylcholine receptor to target rabies (Lentz *et al.,* Science 215: 182 [1982]); Cd3 complement receptor to target Epstein-Barr virus (Carel *et al.,* J. Biol. Chem. 265: 12293 [1990]); β-adrenergic receptor to target reovirus (Co *et al.,* Proc. Natl. Acad. Sci. 82: 1494 [1985]); ICAM-1 (Marlin *et al.,* Nature 344: 70 [1990]), N-CAM, and myelin-associated glycoprotein MAb (Shephey *et al.,* Proc. Natl. Acad. Sci. 85: 7743 [1988]) to target rhinovirus; polio virus receptor to target polio virus (Mendelsohn *et al.,* Cell 56: 855 [1989]); fibroblast growth factor receptor to target herpes virus (Kaner *et al.,* Science 248: 1410 [1990]); oligomannose to target *Escherichia coli;* ganglioside G_{MI} to target *Neisseria meningitidis;* and antibodies to detect a broad variety of pathogens (*e.g., Neisseria gonorrhoeae, V. vulnificus, V. parahaemolyticus, V cholerae,* and *V. alginolyticus).*

### G. Use of Nucleic Acids as Molecular Recognition Elements

In some embodiments of the present invention, the molecular recognition elements are preferably nucleic acids (*e.g.*, RNA or DNA). In some embodiments, the nucleic acid molecular recognition elements are designed to hybridize by base pairing to a particular nucleic acid (*e.g*., chromosomal DNA, mRNA, or ribosomal RNA). In other embodiments, the nucleic acids bind a ligand or biological target. Nucleic acids that bind the following proteins have been identified: reverse transcriptase, Rev and Tat proteins of HIV (Tuerk *et al.,* Gene 137(1):33-9 [1993]); human nerve growth factor (Binkley *et al.,* Nuc. Acids Res. 23(16):3198-205 [1995]); and vascular endothelial growth factor (Jellinek *et al.,* Biochem. 83(34):10450-6 [1994]). Nucleic acids that bind ligands are preferably identified by the SELEX procedure (*See e.g.,* U.S. Pat. Nos. 5,475,096; 5,270,163; and 5,475,096; and in PCT publications WO 97/38134, WO 98/33941, and WO 99/07724) although many methods are known in the art.

### VII. Attachment of Molecular Recognition Elements to Fiberless Radiative Effectors

A variety of methods are be used to attach, fix or conjugate the molecular recognition element or elements to fiberless radiative effectors. In some embodiments, as described above, a copolymer (*e.g*., APMA) comprising a primary amine group is incorporated into the polymer matrix. The amine group serves as a convenient attachment point for molecular recognition elements. In some embodiments, the N-hydroxysuccinimide derivative of biotin is covalently linked to fiberless radiative effector and serves as a universal attachment point for other molecular recognition elements via an avidin linker. In other embodiments, the molecular recognition element is attached to the cloaking material. In still other embodiments, the molecular recognition element is attached to liposomes into which fiberless radiative effectors are loaded.

### A. Biotinylated Fiberless Radiative Effectors

The fiberless radiative effectors of the present invention are covalently bound or attached to molecular recognition elements. In some preferred embodiments, fiberless radiative effectors that serve as universal acceptors for a variety of molecular recognition elements are provided. The fiberless radiative effectors comprise a material that serves as a convenient attachment point for a variety of molecular recognition elements. In some embodiments, the fiberless radiative effectors comprise biotin, which is attached by a variety of methods to either the fiberless radiative effector matrix material or the cloaking material. In other embodiments, the fiberless radiative effectors comprise protein A, anti-mouse IgG, anti-rabbit IgG, or some other protein that recognizes a variety of antibodies.

As described above, in some embodiments of the present invention, fiberless radiative effectors are synthesized that incorporate a copolymer that includes an amine group (*e.g*., APMA). The amine groups provide convenient sites for the covalent attachment of N-hydroxysuccinimide derivatives of biotin. In some preferred embodiments, a N-hydroxysuccinimide derivative of biotin with a spacer arm, preferably a 10 to 15 carbon spacer arm, is utilized ( *e.g.,* biotinamidocaproate N-hydroxysuccinimide ester, biotinamidocaproic acid 3-sulfo-N-hydroxysuccinimide ester, 6-(biotinamidocaproylamido)-caproic acid N-hydroxysuccinimide ester, sulfosuccinimidyl-6-(biotinamido) hexanoate, succinimidyl-6-(biotinamido) *(See e.g.,* Hofmann *et al.,* Biochem. 21:978 [1982]; Lantz and Holmes, Biotechniques 18:58 [1995], Hyman *et al.,* Meth. Enzymol: 196:478 [1991]; Gretch *et al.,* Anal. Biochem. 163:270-77 [1987]). These biotinylated N-hydroxysuccinimide esters react with primary amines, especially aliphatic amines, in aqueous systems at pH 8.0 - 9.0. The aliphatic amide products that are formed are very stable. The inclusion of the spacer arm in the biotinylated N-hydroxysuccinimide esters reduces steric hindrance when binding several biotinylated molecules to one avidin complex.

In some embodiments, other reactive groups in co-polymers serve as attachment site for biotin (*e.g*., biocytin hydrazide, biotin-LC-hydrazide, or biotin hydrazide are used to biotinylate aldehyde groups in carbohydrates; N-(6-[biotinamido]hexyl)-3'-(2'-pyridylthio)propionamide, 1-biotinamido-4-[4'(maleimidomethyl)-cyclohexane-carboxamido butane, 3-(N-maleimido-propionyl) biocytin (Bayer *et al.,* Anal. Biochem. 149:529-36 [1985]) or N-iodoacetyl-N-biotinyl hexylene diamine are used to biotinylate materials with thiol groups).

In other embodiments of the present invention, the cloaking agent is biotinylated with a suitable biotin derivative (*e.g*., a N-hydroxysuccinimide derivative of biotin with a spacer arm is used to biotinylate fiberless radiative effectors cloaked with amino-PEG; biocytin hydrazide, biotin-LC-hydrazide, or biotin hydrazide are used to biotynylate fiberless radiative effectors cloaked with COOH-PEG). In some embodiments, biotinylated PEG is used as the cloaking material (*See e.g.,* Kaiser *et al.,* Bioconj. Chem. 8(4):545-51 [1997]) In still other embodiments, the PEG coat of a liposome containing fiberless radiative effectors is biotinylated as above.

In some preferred embodiments of the present invention, peptide or protein molecular recognition elements (*e.g.,* antigen binding proteins, antibodies, RGD peptides, IL13 and its variants, or carbohydrates such as sialic acid) are also biotinylated by selecting an appropriate reagent as described above. In other preferred embodiments, nucleic acid molecular recognition elements are synthesized with biotinylated base (*See e.g.,* Leary *et al.,* PNAS 80: 4045-49 [1983]; Langer *et al.,* PNAS 78:6633-37 [1981]; Brigati *et al.* Virology 126: 32-50 [1983]), or biotinylated bases are added to 3' or 5' terminal base with a terminal transferase, *Taq* polymerase, or poly(A)polymerase *(See e.g.,* Sambrook *et al., Molecular Cloning: A Laboratory Manual,* Cold Spring Harbor Press, NY, pp 9.31-9.58 [1989]).

In still further embodiments of the present invention, the biotinylated molecular recognition element is linked to the biotinylated fiberless radiative effector (*i.e*., biotinylated either directly or on the cloaking material) with avidin. Avidin is a glycoprotein found in egg white and contains four identical subunits having a combined molecular mass of 67 - 68 kD. Each subunit binds one molecule of biotin, therefore, combining the avidin, biotinylated fiberless radiative effector, and biotinylated molecular recognition element results in the formation of a fiberless radiative effector-biotin-avidin-biotin-molecular recognition element complex that binds to a biological target.

### B. Direct Cross-Linking of Molecular Recognition Elements to Fiberless Radiative Effectors and Cloaking Materials

In some embodiments of the present invention, molecular recognition elements containing amine groups (*e.g*., antigen binding proteins, antibodies, RGD peptides, or IL13 and its variants) are crosslinked with the fiberless radiative effector via a homobifunctional crosslinking agent or heterobifunctional cross-linking agent. In some preferred embodiments, the crosslinking agent includes a linker arm so that steric interference does not hinder binding of the molecular recognition element to its biological target. In other preferred embodiments, a crosslinking agent is chosen that does not alter the binding specificity of the molecular recognition element *(See e.g.,* Brinkley, Bioconj. Chem. 3:2-13 [1992]; Zapalinsky, Bioconj. Chem.6:150-65 [1995]. In some preferred embodiments, the sequence CDCRGDCFC (*i.e.,* RGD peptide; SEQ ID NO:1) is conjugated using HISTAG or succinimidyl ester to primary amine linkers.

In some preferred embodiments, the fiberless radiative effectors include a co-polymer with a primary amine group (*e.g.,* APMA, acryloamidodextran, aminomethylstyrene, 2-hydroxyethyl acrylate, 2-hydroxymethacrylate, 4-hydroxybutyl acrylate; *See e.g.,* Daubresse *et al.,* J. Pharm. Pharmacol. 45(12):1018-23 [1993]; Noguchi *et al.,* J. Biomed. Mat. Res. 39(4):621-9 [1998]). In some preferred embodiments, the copolymer (*e.g.,* APMA) is itself partially functionalized and binds the molecular recognition element directly. In other embodiments, the fiberless radiative effectors are crosslinked to molecular recognition elements that contain amine groups via a homobifunctional crosslinking agent that is reactive towards amine groups (*e.g.,* disuccinimidyl suberate, bis(sulfosuccinymidyl)suberate, disuccinimydal glutarate, dimethyladipimidate-2HCl, or dimethylpimelimidate-2HCl). In other embodiments, the amine containing fiberless radiative effectors are cross-linked to proteins containing thiol groups via a heterobifunctional crosslinker that is reactive towards thiols and primary amines (*e.g.,* succinimydyl 3-(2-pyridyldithio)propionate (SPDP); succinimidyl trans-4-(N-maleimidylmethyl)cyclohexane-1-carboxylate (SMCC); succinimidyl (acetylthio)acetate (SATA); 4-[(succinimydyloxy)carboxyl]-α-methyl-α-(2-pyridyldithio)toluene (SMPT); succinimidyl 4-[(iodoacety10amino]methyl]-cyclohexane-1-carboxylate (SIAC); m-maleimidobenzoyl-N-hydroxysuccinimide ester (MBS), or succinimydyl 4-(p-maleimdophenyl)-butyrate (SMPB)). In still other embodiments, the amine containing fiberless radiative effector is crosslinked to a glycoprotein (*i.e.,* carbohydrate containing protein) or carbohydrate via a heterobifunctional crosslinking reagent that is reactive towards both carbohydrates and primary amines or thiols (*e.g*., 4-[N-Maleimidomethyl]cyclohexane-1-carboxylhyorazide-HCl-1/2 dioxane, 4-[4-Maleimidophenyl]butyric acid hydrazide-HCl). In other embodiments, the peptide molecular recognition elements are directly cross-linked to the cloaking material by choosing an appropriate bifunctional cross-linking agent as described above. In other embodiments, the co-polymer is preferably a semitelechelic poly[N-(2-hydroxypropyl)methacrylamide] with a functional end group (*e.g.,* containing a carboxyl, methyl ester, hydrazide, or amino group) *(See. e.g.,* Lu *et al.,* Bioconj. Chem. 9(6):793-804 [1998]). In still other embodiments, the co-polymer is preferably a poly(amidoamino) formed by addition of amines to bisacrylamide *(See e.g.,* Ferruti *et al.,* Biomaterials 15(15):1235-41 [1994]). In still other embodiments, the co-polymer is preferably polypropylene functionalized with primary amino groups *(See e.g.,* Das and Lindstrom, Biochem. 30(9):2470-7 [1991]; Geysen, Immunol. Meth. 102:259-74 91987]).

In some embodiments, functionalized derivatives of poly(ethylene glycol) allow convenient attachment of molecular recognition elements to the fiberless radiative effector *(See e.g.,* Zalipsky, Bioconj. Chem. 495):314-18 [1993]). In some preferred embodiments, the functionalized poly(ethylene glycol) is used as either a co-polymer or as a cloaking material for the fiberless radiative effector. In some embodiments, liposomes or fiberless radiative effectors are prepared (*i.e.,* cloaked) from monomethoxypoly(ethylene glycol), to which oligonucleotides and peptides are conjugated (*See e.g.,* Bonora *et al*., Farmaco 53:634-7 [1998]; Cheng *et al.,* Cancer Immunol. Immunother. 44(6):305-15 [1997]). In other embodiments, peptide molecular recognition elements may be conjugated to polyethyleneglycol acetaldehyde by reductive amination *(See e.g.,* Bentley *et al.,* J. Pharm. Sci. 87( 11):1446-9 [1998]). In other embodiments, the cloaking material is preferably a N-hydroxysuccinimide ester of polyethylene glycol to which molecular recognitions elements containing primary amine groups are attached *(See e.g.,* Clark *et al.,* J. Biol. Chem. 271(36)21969-77 [1996]). In still other embodiments, the amino terminal serine or threonine of some peptides is modified on the N-terminal by periodate oxidation to form reactive carbonyl group (e.g., glyoxylyl group). The N-terminal carbonyl group is then reacted with aminooxy-functionalized poly(ethylene glycol) (Gaertner and Offord, Bioconj. Chem. 7(1):38-44 [1996]). In some embodiments, the peptides not normally having a N-terminal serine or threonine are modified to contain one or the other. In still other embodiments, a thiol-protected poly(ethylene glycol) intermediary that reacts with cysteine residues in peptides is preferably produced from monomethoxy poly(ethylene glycol)(Woghiren *et al.,* Bioconj. Chem. 4(5):314-18 [1993]). In some embodiments, liposomes or fiberless radiative effectors are prepared (*i.e.,* cloaked) with a dipalmitoylphosphatidylethanolamine derivative of PEG with a terminal maleimidyl group to which thiol containing compounds (*e.g.*, antibodies) are conjugated *(See e.g.,* Maruyama *et al.,* FEBS Letters 413:177-180 [1997]).

In still further embodiments, the fiberless radiative effector comprises a polymer matrix containing carbohydrate, sulfhydryl, or some other functionalizable group. An appropriate heterobifunctional or homobifunctional crosslinking reagent is selected as described above and used to covalently attach the fiberless radiative effector to the peptide molecular recognition element. In still further embodiments, molecular recognition elements containing carbohydrate groups by crosslinked directly to the fiberless radiative effector matrix or cloaking material as described.

In some embodiments, two or more molecular recognition elements are attached to the fiberless radiative effector or cloaking materials. For example, in some embodiments, the fiberless radiative effector is biotinylated and crosslinked to another molecular recognition element (*e.g*., RGD peptide, IL13, or an antigen binding protein) as described above. In other embodiments, the fiberless radiative effector is crosslinked or attached to avidin and some other peptide or protein.

Attachment of more than two molecular recognition elements to a fiberless radiative effector allows their utilization in various stacking approaches. For example, in some embodiments, a first fiberless radiative effector includes a first molecular recognition element (*e.g*., antigen binding protein, RGD peptide, IL13, or sialic acid), a second molecular recognition element (*e.g*., biotin), and a radiodynamic compound. A second fiberless radiative effector includes a gamma ray emitter and is crosslinked to avidin. The first fiberless radiative effector binds to a biological target (i.e., pathogen or tumor cell) via the first molecular recognition element, and the second fiberless radiative effector binds to the first fiberless radiative effector via avidin-biotin binding to form a biological target-first fiberless radiative effector-biotin-avidin-second fiberless radiative effector complex. The gamma-rays emitted from the second fiberless radiative effector excite the radiodynamic compound resulting in the production of singlet oxygen and free radicals. As another example, in some embodiments, a first fiberless radiative effector includes a first molecular recognition element (*e.g*., antigen binding protein, RGD peptide, IL13, or sialic acid), a second molecular recognition element (*e.g*., biotin), and contains a photodynamic compound. A second fiberless radiative effector includes an enzyme that catalyzes a bioluminescent reaction (*e.g*., bacterial or insect luciferase) and is crosslinked to avidin. The first fiberless radiative effector binds to a biological target (*i.e*., pathogen or tumor cell) via the first molecular recognition element, and the second fiberless radiative effector binds to the first fiberless radiative effector via avidin-biotin binding. Photons produced by the action of the enzyme on a substrate (e.g., luciferin) excite the photodynamic compound in the first fiberless radiative effector so that singlet oxygen and free radicals are produced.

### C. Linkage of Other Toxic Agents to Fiberless Radiative Effectors

The present invention also contemplates that a variety of additional toxic agents may be conjugated to or incorporated into the fiberless radiative effector, cloaking material, or liposome as described above. Such toxic agents may be utilized in fiberless radiative effectors in conjunction with, for example, photodynamic compounds or radiodynamic compounds, or by themselves as the primary toxic agent. Examples of such toxic agents and the necessary linkage are given in Table 1.

**Table 1**

| | **Toxic Agents** | |
|---|---|---|
| **Name** | **Linkage** | **Source** |
| **Antimicrobials** | | |
| Streptomycin | ester/amide | |
| Neomycin | ester/amide | Dow, Lilly |
| Amikacin | ester | Bristol |
| Gentamicin | ester/amide | Upjohn |
| Tobramycin | ester/amide | Lilly |
| Streptomycin B | ester/amide | Squibb |
| Spectinomycin | ester | Upjohn |
| Ampicillin | amide | Squibb |
| Sulfaniliamide | amide | Merrell-National |
| Polymixin | amide | Burroughs-Wellcome |
| Chloramphenicol | ester | Parke-Davis |
| | **Antivirals** | |
| Acyclovir | | Burroughs-Wellcome |
| Vira A | ester/amide | Parke-Davis |
| Symmetrel | amide | Endo |

| | **Antifungals** | |
|---|---|---|
| Nystatin | ester | Squibb |
| | **Antineoplastics** | |
| Adriamycin | ester/amide | Adria |
| Cerubidine | ester/amide | Ives |
| Bleomycin | ester/amide | Bristol |
| Alkeran | amide | Burroughs-Wellcome |
| Velban | ester | Lilly |
| Oncovin | ester | Lilly |
| Fluorouracil | ester | Adria |
| Methotrexate | amide | Lederic |
| Thiotepa | | Lederic |
| Bisantrene | | Lederic |
| Novantrone | ester | Lederic |
| Thioguanine | amide | Burroughs-Wellcome |
| Procarabizine | | Hoffman-La Roche |
| Cytarabine | | Upjohn |
| | **Heavy Metals** | |
| Barium | | |
| Gold | | |
| Platinum | | |

| **Antimycoplasmals** | | |
|---|---|---|
| Tylosine | | |
| Spectinomycin | | |

### VIII. Uses of Fiberless Radiative Effectors

The fiberless radiative effectors of the present invention are utilized in a variety of *in vivo* and *in vitro* procedures. In some embodiments, the fiberless radiative effectors are utilized to destroy or inhibit the growth of a biological target (*e.g*., pathogens, macromolecules, or tumor cells in culture or in the body). In other embodiments, the fiberless radiative effectors are used as markers (*e.g.*, fluorescent markers or magnetic markers) for the detection of a biological target.

### A. Use for Photodynamic and Radiodynamic Therapy of Tumors

In some embodiments of the present invention, fiberless radiative effectors comprising molecular recognition elements that specifically bind to tumor cells (*e.g*., gliomas or squamous cancer cells) are used to treat patients with cancer. A variety of molecular recognition elements are attached to such fiberless radiative effectors. In some embodiments, the molecular recognition element is a peptide or protein (*e.g.,* RGD peptide or IL13) that acts as a ligand for a tumor cell specific protein (*e.g*., cell membrane receptor, extracellular matrix protein). In other embodiments, the molecular recognition elements is an antigen binding protein (*e.g.,* antibody, single chain antibody, or Fab fragment) that binds a tumor cell specific antigen. Examples of such antigen binding proteins and antigens are described above.

### 1. Malignant Glioma

Among the causes of death due to cancer, brain tumors are ranked second in the pediatric age group and fourth in middle-aged men. Despite the use of multimodality therapy, malignant gliomas are uniformly fatal; only 50% of patients survive even one year from the time of diagnosis. These stark statistics underscore the urgent need for novel therapeutic strategies and delivery vehicles for the treatment of these tumors.

Primary intracranial neoplasms occur at a rate of 4.5-8.2 per 100,000 people per year. In the United States, an estimated 9,000 to 17,000 new primary tumors are diagnosed each year. Despite recent advances in neurosurgery, neuroradiology, and neurooncology, the multimodality approach currently used in the treatment of malignant brain tumors produces few cures. Malignant gliomas constitute a major clinical problem because of their frequency of occurrence and extremely poor prognosis. Although neurosurgical resection can remove 90% of a malignant astrocytoma, a residual tumor may still contain 10¹⁰ cells. Radiation and chemotherapy are typically given following surgical resection yet the median life span remains less than 1 year with fewer than 20% of patients surviving 2 years from initial diagnosis. Immunotherapy has also proven to be inadequate due to the relatively large numbers of glioma cells surviving initial immunological responses. Although many technical obstacles have been overcome, the most recent strategies, including the use of hyperthermia in the treatment of these tumors, have proven unfruitful in extending the life expectancy of patients with malignant gliomas. Although there are numerous reasons for the lack of significant progress in the treatment of many cranial tumors, the unfavorable results obtained to date in the treatment of malignant astrocytomas underscores the need for developing and investigating novel and aggressive therapeutic approaches to treat these diseases.

### 2. Head and Neck Cancer

Head and neck cancer is any type of cancer of the upper airways/digestive tract, face, or neck. In 1995, about 50,000 head and neck cancers were expected to be diagnosed in this country. Over 13,000 people were expected to die from the condition. Head and neck cancers are three times more common in men than in women. Over 90 percent of head and neck cancers are squamous cell carcinomas. Excessive use of alcohol or tobacco is the most common risk factor for the disease and is associated with over 95 percent of cases. Use of both alcohol and tobacco (including smokeless tobacco) significantly increases risk for the condition. Exposure to toxic fumes and chemicals may predispose an individual to cancers of the oral cavity and throat.

Two-thirds of all patients with squamous cell carcinoma of the head and neck (SCCHN) present with locally advanced disease. For these patients, the treatment offering the best chance of local control/cure consists of surgery (which is often disfiguring and functionally morbid) followed by postoperative radiotherapy (RT). For patients with medically inoperable or unresectable locally advanced disease, radiotherapy alone is the standard treatment; however, results of such treatment are unsatisfying with 5 year survival rates of less than 25% and most treatment failures occurring locally or regionally (*i.e*., within the irradiated fields). Since only 10% of patients with SCCHN develop metastatic disease, it is clear that the development of improved non-surgical treatments for locally advanced disease would represent a significant advance in the treatment of unresectable/inoperable locally advanced SCCHN and might provide less morbid, organ-preserving options for patients with resectable disease. The combination of chemotherapy and RT has been extensively studied as a strategy to improve the results of non-surgical therapy for SCCHN. Unfortunately, despite promising responses to the concurrent administration of chemotherapy and radiation, the clinical utility of concurrent chemotherapy and radiation as the initial definitive non-surgical management of locally advanced SCCHN remains limited by the increased toxicity (mucosal as well as systemic) of the combined treatments.

### 3. Treatment of Malignant Glioma and Head and Neck Cancer

In some preferred embodiments, fiberless radiative effectors with IL13 molecular recognition elements are used to treat patients who have malignant gliomas. In some embodiments, the fiberless radiative effector for treatment of glioma comprises a polymer matrix (e.g., acrylamide or decylacrylate) including a toxic agent, preferably a photodynamic compound (e.g., ruthenium red or Photofrin II), a cloaking material (*e.g.*, PEG or a liposome) and IL13 attached to either the cloaking material or the polymer matrix (*e.g.*, via a heterobifunctional or homobifunctional crosslinker or biotin as described above). In some embodiments, the fiberless radiative effectors administered to the patient intravenously. The fiberless radiative effectors are then allowed to bind to the tumor cells. In some embodiments, the fiberless radiative effectors are illuminated (*e.g.*, by fiber optics or a laser, or combination thereof) so that the photodynamic compound is excited, resulting in the production of singlet oxygen and free radicals (*e.g*., superoxide or hydroxyl radicals). In some preferred embodiments, the singlet oxygen and free radicals diffuse from the fiberless radiative effector into the tumor cells, resulting in tumor necrosis. In other preferred embodiments, head and neck cancer is treated as described, except that the RGD peptide is utilized as the molecular recognition element.

In other preferred embodiments, the fiberless radiative effector for treatment of glioma comprises a polymer matrix (*e.g*., poly(styrene)) including a toxic agent, preferably a radiodynamic compound (*e.g*., 2,5-diphenyloxazole (PPO)), a photodynamic compound (*e.g.*, Photofrin II), a cloaking material (*e.g.,* PEG or a liposome) and IL13 attached to either the cloaking material or the polymer matrix (*e.g.,* via a heterobifunctional or homobifunctional crosslinker or biotin). In some embodiments, the fiberless radiative effectors administered to the patient intravenously. The fiberless radiative effectors are then allowed to bind to the tumor cells. In some embodiments, the fiberless radiative effectors are exposed to X-rays so that energy (*i.e.,* excitons) is transferred to the photodynamic compound, resulting in the production of singlet oxygen and free radicals (*e.g*., superoxide or hydroxyl radicals). In some preferred embodiments, the singlet oxygen and free radicals diffuse from the fiberless radiative effector into the tumor cells, resulting in tumor necrosis. In other preferred embodiments, head and neck cancer is treated as described, except that the RGD peptide is utilized as the molecular recognition element.

In other preferred embodiments, the fiberless radiative effector for treatment of glioma comprises a polymer matrix (*e.g*., poly(styrene)) including a toxic agent, preferably a radiodynamic compound (*e.g*., 2,5-diphenyloxazole (PPO)), a photodynamic compound (*e.g*., Photofrin II), a cloaking material (*e.g*., PEG or a liposome); IL13 attached to either the cloaking material or the polymer matrix (*e.g*., via a heterobifunctional or homobifunctional crosslinker or biotin), and biotin attached to either the cloaking material or polymer matrix. In some embodiments, the fiberless radiative effectors administered to the patient intravenously. The fiberless radiative effectors are then allowed to bind to the tumor cells. In some embodiments, a second fiberless radiative effector is provided that comprises a polymer matrix including a gamma ray emitter, cloaking material, and avidin attached to either the polymer matrix or the cloaking material. In some embodiments, the second fiberless radiative effectors are administered intravenously so that they bind to the first fiberless radiative effectors. The first fiberless radiative effectors are exposed to gamma-rays so that energy (*i.e.,* excitons) is transferred from the radiodynamic compound to the photodynamic compound, resulting in the production of singlet oxygen and free radicals (*e.g*., superoxide or hydroxyl radicals). In some preferred embodiments, the singlet oxygen and free radicals diffuse from the fiberless radiative effector into the tumor cells, resulting in tumor necrosis. In other preferred embodiments, head and neck cancer is treated as described, except that the RGD peptide is utilized as the molecular recognition element.

### B. Use in Photodynamic Therapy of Pathogen Exposure

In some embodiments of the present invention, fiberless radiative effectors comprising molecular recognition elements that specifically bind to pathogens *(e.g., Legionella peomophilia, Mycobacterium tuberculosis, Clostridium tetani, Hemophilus influenzae, Neisseria gonorrhoeae, Treponema pallidum, Bacillus anthracis, Vibrio cholerae, Borrelia burgdorferi, Cornebacterium diphtheria, Staphylococcus aureus,* human papilloma virus, human immunodeficiency virus, rubella virus, polio virus) are used to treat patients who have been exposed to the pathogen. In some embodiments, fiberless radiative effectors comprise a polymeric matrix, a photodynamic compound, a cloaking agent, and a molecular recognition element that binds to a pathogen epitope (described in detailed above). In some embodiments, a patient who has been exposed to a pathogen is treated by intravenous administration of a fiberless radiative effector that recognizes the pathogen. In some embodiments, about 10⁸ to 10¹⁸ (each about 50 nm), most preferably about 10¹⁴ fiberless radiative effectors, are administered (*e.g*., orally or intravenously) to the patient. In some embodiments, each fiberless radiative effector contains about 0.1 to 10% of the photodynamic compound, preferably about 1.0% of the photodynamic compound. In other embodiments, about 1 µg/kg to 1000 µg/kg, most preferably about 100 µg/kg, fiberless radiative effectors are administered. In some embodiments, the fiberless radiative effectors are then allowed a period of time to bind the pathogen, preferably about 1 minute to 24 hours, most preferably for about an hour, resulting in the formation of a pathogen-fiberless radiative effector complex. In some embodiments, the fiberless radiative effectors are then illuminated (*e.g*., with a red laser, incandescent lamp, or filtered sunlight). In some embodiments, the light is aimed at the jugular vein or some other superficial blood or lymphatic vessel. In some embodiments, the singlet oxygen and free radicals diffuse from the fiberless radiative effector to the pathogen, causing its destruction.

In other embodiments, fiberless radiative effectors comprise a polymeric matrix, a radiodynamic compound, a photodynamic compound, a cloaking agent, and a molecular recognition element that binds to a pathogen epitope (described in detailed above). In some embodiments, a patient who has been exposed to a pathogen is treated by intravenous administration of a fiberless radiative effector that recognizes the pathogen. In some preferred embodiments, about 10⁸ to 10¹⁸ (each about 50 nm), most preferably about 10¹⁴ fiberless radiative effectors, are administered to the patient. In some embodiments, each fiberless radiative effector contains about 0.1 to 10% of the photodynamic compound, preferably about 1.0% of the photodynamic compound. In other embodiments, about 1 µg/kg to 1000 µg/kg, most preferably about 100 µg/kg, fiberless radiative effectors are administered. In some embodiments, the fiberless radiative effectors are then allowed a period of time to bind the pathogen, preferably about 1 minute to 24 hours, most preferably for about an hour, resulting in the formation of a pathogen-fiberless radiative effector complex. In some embodiments, the fiberless radiative effectors are then exposed to X-rays, resulting in the production of singlet oxygen and free radicals as described above. In some embodiments, the singlet oxygen and free radicals diffuse from the fiberless radiative effector to the pathogen, causing its destruction.

In still other embodiments, fiberless radiative effectors comprise a polymeric matrix, a radiodynamic compound, a photodynamic compound, a cloaking agent, biotin and a molecular recognition element that binds to a pathogen epitope (described in detailed above). In some embodiments, a patient who has been exposed to a pathogen is treated by intravenous administration of a fiberless radiative effector that recognizes the pathogen. In some preferred embodiments, about 10⁸ to 10¹⁸ (each about 50 nm), most preferably about 10¹⁴ fiberless radiative effectors, are administered to the patient. In some embodiments, each fiberless radiative effector contains about 0.1 to 10% of the photodynamic compound, preferably about 1.0% of the photodynamic compound. In other embodiments, about 1 µg/kg to 1000 µg/kg, most preferably about 100 µg/kg, fiberless radiative effectors are administered. In some embodiments, the fiberless radiative effectors are then allowed a period of time to bind the pathogen, preferably about 1 minute to 24 hours, most preferably for about an hour, resulting in the formation of a pathogen-fiberless radiative effector complex. In some embodiments, a second fiberless radiative effector is provided comprising a polymeric matrix, a gamma ray emitter, a cloaking agent, and avidin. The second fiberless radiative effector is administered intravenously at similar concentration as the first fiberless radiative effector. In some embodiments, the second fiberless radiative effector binds to the first fiberless radiative effector, thereby exposing the first fiberless radiative effector radiodynamic compound to gamma rays. In some embodiments, energy (*i.e*., excitons) is transferred from the radiodynamic compound to the photodynamic compound, causing the excitation of the photodynamic compound and production of singlet oxygen and radicals. In some embodiments, the singlet oxygen and free radicals diffuse from the fiberless radiative effector to the pathogen, causing its destruction.

### C. Use of Fiberless Radiative Effectors for Imaging

In some embodiments of the present invention, the fiberless radiative effectors are used for imaging or detecting a biological target. In some preferred embodiments, a signaling material (*e.g*., magnetic particle, fluorescent compound, or bioluminescent compound) or other detectable material is incorporated into the fiberless radiative effector polymeric matrix. It is desirable that the material is easily detectable at low levels so that the imaging is conducted in *situ.* In this manner, the response of a biological target (*e.g*., tumors) to treatment can be assayed. A major advantage of *in situ,* non-invasive measures of tumor response is that a single test subject (*e.g*., mouse or other animal) can be followed with time. Therefore, tumor volumes can be measured without sacrificing the animal, resulting in the use of fewer animals. *In situ* imaging is also be used to follow tumor regression following treatment in humans. In some embodiments, the detectable material is selected from magnetic materials (*e.g*., iron for MRI); proteins that catalyze luminescent reactions (*e.g.*, luciferase for bioluminescent imaging); fluorescent dyes (*e.g*., rodamine or fluorescein isothiocyanate for fluorescent imaging); fluorescent proteins (*e.g*., green fluorescent protein); and radioactive elements (*e.g.*, for autoradiography).

### 1. Bioluminescent Imaging

In some embodiments, fiberless radiative effectors of the present invention are used to detect biological targets *in vivo* or in *vitro* by bioluminescent imaging. In some embodiments, fiberless radiative effectors comprise a polymeric matrix (*e.g.,* sol-gel or acrlyamide), an enzyme that catalyzes a bioluminescent reaction, a cloaking agent, and a molecular recognition element (*e.g.,* antigen binding protein, RGD peptide or IL13) that binds to a biological target (*e.g.,* tumor cells, described in detailed above). In some preferred embodiments, the matrix material allows the diffusion of substrates (*e.g*., luciferin and ATP) to the enzyme. Enzymes that catalyze bioluminescent reactions include the following luciferases: bacterial luciferase (U.S. Pat. No. 4,548,994), *Photinus pyralis* luciferase (U.S. Pat. Nos. 5,670,356 and 5,674,713), *Renilla reniformus* luciferase, *Pyrophorus plagiophihalamus* luciferase, *Luciola cruciata* luciferase (Masuda *et al.,* Gene 77:265-70 [1989]), *Luciola lateralis* luciferase (Tatsumi *et al.,* Biochim. Biophys Acta 1131:161-65 [1992]), and *Latia neritoides* luciferase.

In some embodiments, the imaging is performed *in situ* (*See e.g.,* Contag *et al.,* Nature Med. 4(2):245-47 [1998]. Fiberless radiative effectors containing the bioluminescent enzyme are provided to the animal intravenously and allowed time so that the molecular recognition element binds to its biological target. In some embodiments, a substrate (*e.g*., bacterial or insect luciferin) for the bioluminescent enzyme is then provided (*e.g.,* via intravenous, intraperitoneal, intravesical, or intracerebrovascular delivery) to the animal. In some embodiments, production of bioluminescence by the action of the enzyme on the substrate is then detected by a bioluminescence detection system. In some embodiments, the bioluminescence detection system comprises a Hamamatsu intensified CCD (ICCD, model C2400-32). In other embodiments, the bioluminescence detection system further comprises other devices for intensifying weak signals (*e.g.,* microchannel plate intensifiers and devices for Peltier or liquid nitrogen cooling of the detector and/or intensifier). In some preferred embodiments, a grey scale image of the animal is obtained by opening the door of dark chamber in which the animal is placed. The door is then shut and the gain on the intensifier adjusted to maximum to detect the bioluminescent signal. The signal is then overlaid with the greyscale image in pseudocolor.

The use of fiberless radiative effectors targeted to a particular biological target has advantages over previously described methods of *in situ* bioluminescent imaging (*See e.g.,* Contag *et al., supra*). The previously described methods require injection of tumor cells that express luciferase into mice. Obviously, this approach can not be transferred to the clinical environment. In contrast, fiberless radiative effectors are non-toxic, can be designed to image virtually any biological target, and can be used in clinical setting.

### 2. Magnetic Resonance Imaging

In other preferred embodiments, fiberless radiative effectors of the present invention are used to detect biological targets by magnetic resonance imaging (MRI). In some embodiments, fiberless radiative effectors comprise a polymeric matrix (*e.g*., sol-gel or acrlyamide), a magnetic material (*e.g*., iron oxide), a cloaking agent, and a molecular recognition element (*e.g*., antigen binding protein, RGD peptide or IL13) that binds to a biological target (*e.g*., tumor cells, described in detailed above). In some embodiments, the fiberless radiative effector also comprises a toxic agent, so that the fiberless radiative effector is utilized to both destroy or inhibit the growth of a biological target and image the biological target. Accordingly, the fiberless radiative effector can be used to both monitor tumor growth and response to therapy.

In some embodiments, the biological target imaged is *in situ* (*See e.g.,* Ross *et al.,* PNAS 95:7012-17 [1998]). Fiberless radiative effectors containing the magnetic material are provided to the animal (*e.g.,* intravenously) and time allowed so that the molecular recognition element binds to its biological target. In some embodiments, the biological target is then imaged with a magnetic resonance system (*e.g*., a 7-Tesla Magnetic Resonance System). In some embodiments, T1-weighted or T2-weighted images are obtained.

In some embodiments, it is contemplated that inclusion of the magnetic material in the fiberless radiative effector enhances photodynamic therapy.

It has been shown recently that the photohemolysis of human erythrocytes with the non-steroidal antiinflammatory agent ketoprofen is dramatically amplified by either a static external magnetic field (Chignell and Sik, Photochem. Photobiol. 67:591-95 [1998]; Chignell and Sik, Photochem. Photobiol. 62:205-7 [1995]) or the fields due to the presence of magnetic nanoparticles (Chignell and Sik, Photochem. Photobiol. 68:598-601 [1998]; Scaiano *et al.,* Photochem. Photobiol. 65:759-62 [1997]). The hypothesized mechanism (Scaiano *et al.,* Photochem. Photobiol. 59:585-89 [1994]) involves the formation of triplet radical pairs in the ketoprofen reaction with cell lipids, which often undergo an intersystem crossing to a singlet pair that can react with itself, simply replenishing the initial reactants. In the presence of the magnetic fields, though, the triplet/singlet degeneracy is broken in the radical pair, causing a slowdown in the intersystem crossing rate to self-annihilating singlet pairs, and a concomitant increase in the concentration and/or lifetime of free radicals that escape from the pair, and are thus available for further reactions causing cell damage.

### D. Use for Removing Sub-population of Cells from Mixture

In some embodiments of the present invention, the fiberless radiative effectors are used to eliminate a subpopulation of cells from a mixture of cells, thereby creating a purified cell population from the remaining cells. In some embodiments, T-cells may be removed from a sample containing a mixture of T-cells and B-cells. In some preferred embodiments, there is provided a sample containing a mixture of B-cells and T-cells and fiberless radiative effectors which recognize or are specific for T-cells. In some embodiments, the fiberless radiative effectors comprise a toxic agent and molecular recognition element which specifically recognizes T-cells. In other embodiments, the fiberless radiative effectors and cell sample are combined so that the fiberless radiative effectors bind to T-cells contained in the sample. In still further embodiments, the fiberless radiative effectors are illuminated so that free-radicals are produced, resulting in the depletion of T-cells from the sample.

In some embodiments, the molecular recognition element that specifically recognizes T-cells is the monoclonal antibody OKT3 (ATCC CRL 8001; See, *e.g.,* U.S. Pat. No. 4,731,244). In other embodiments, the molecular recognition element comprises an anti-TAC antibody (See, *e.g.,* Kupiec-Weglinski et al., PNAS 83:2624 [1986]) or another antibody that recognizes T-cells *(See, e.g.,* Abdi *et al.,* J. Immunol. 142(9):2971-80 [1989] and *Wang et al.,* Eur. J. Immunol. 24(7):1549-52 [1994]).

### IX. Other Preparations for In Vivo Use

The present invention contemplates using therapeutic compositions of fiberless radiative effectors, in particular for treatment of cancer and exposure to pathogens. For example, such compositions can be provided together with physiologically tolerable liquid, gel or solid carriers, diluents, adjuvants and excipients. In addition, the fiberless radiative effectors are used with other therapies, including chemotherapy and radiation therapy for the treatment of cancer, and antibiotic and vaccine therapy for treatment for exposure to pathogens.

With respect to the mode of administration, methods for administration of the fiberless radiative effectors include intravenous, intramuscular, intrathecal or topical (including topical ophthalmic) administration. Formulations for such administrations comprise an effective amount of fiberless radiative effectors analog in sterile water or physiological saline.

On the other hand, some formulations contain such normally employed additives as binders, fillers, carriers, preservatives, stabilizing agents, emulsifiers, buffers and excipients as, for example, pharmaceutical grades of mannitol, lactose, starch, magnesium stearate, sodium saccharin, cellulose, magnesium carbonate. These compositions typically contain 1%-95% of active ingredient, preferably 2%-70%.

The compositions are preferably prepared as injectables, either as liquid solutions or suspensions; solid forms suitable for solution in, or suspension in, liquid prior to injection may also be prepared.

The fiberless radiative effectors of the present invention are often mixed with diluents or excipients that are compatible and physiologically tolerable. Suitable diluents and excipients are, for example, water, saline, dextrose, glycerol and combinations thereof. In addition, if desired the compositions may contain minor amounts of auxiliary substances such as wetting or emulsifying agents, stabilizing or pH buffering agents.

Likewise, dosage ranges for fiberless radiative effector treatment include . 1 ng/kg/day to 200 mg/kg/day.

### EXPERIMENTAL

The following examples are provided in order to demonstrate and further illustrate certain preferred embodiments and aspects of the present invention.

### Example 1

### Synthesis of PEG-Cloaked Acrlyamide Fiberless Radiative Effector Containing Ruthenium Dye and Cross-Linked to RGD Peptide

This example describes the synthesis of fiberless radiative effectors comprising an acrylamide core, a RGD peptide molecular recognition element, a photodynamic compound, and PEG cloaking material.

Preparation of Acrylamide Nanospheres: The polymerization solution was made by combining 50 mg ruthenium dye, 27% acrylamide, 3% N,N-methylenebis(acrylamide), in 1 mL of 10 mM phosphate buffer, pH 7.4. The polymerization solution was then added to a solution containing 20 mL hexane, 1.8 mmol dioctyl sulfosuccinate sodium salt and 4.24 mmol Brij 30 (4 Lauryl ether), and the two solutions were emulsified by stirring. The polymerization was initiated with 50 µL of a 10% sodium bisulfite solution OR 24 µL of a 10% ammonium persulfate solution and 12 µL N,N,N,N-tetramethylethylenediamine (TEMED), and the solution was stirred at room temperature for 2 hours. Hexane was removed by rotary evaporation and then the effectors were precipitated by the addition of ethanol. Excess surfactant and dye were removed by rinsing with ethanol, to yield a product consisting of 20 and 200 nm probes.

PEG Cloaking: Nanospheress are diluted in deionized water to 0.3 % (w/v), then added to an equal volume of -hydroxy PEG (2 % w/v) (Aldrich, Milwaukee, WI) in water and incubated overnight at room temperature.

RGD crosslinking: Fiberless radiative effectors are produced by stirring the PEG cloaked nanospheres with NHS-biotin and triethylamine in dichloromethane and acetonitrile at room temperature under nitrogen overnight. The fiberless radiative effectors are then isolated by vacuum filtration and dried from toluene azeotrope. The biotinylated fiberless radiative effectors are then incubated with avidin linked RGD, and incubated for three hours at room temperature. The final product is rinsed and filtered with phosphate buffer.

### Example 2

### Synthesis of Decyl-Methacrylate Fiberless Radiative Effector Cross-Linked to IL13

Preparation of Cloaked Decyl-methacrylate Nanospheres: To produce the nanospheres, HCl is placed in a two necked, 100ml round bottom flask and nitrogen is slowly run through the flask while the solution is stirring. A cloaking agent, oxidized dextran (also called side-on dextran) (440 mg) is then added to the flask. The decyl methacrylate (55 mg), hexanedioldimethacrylate (75 mg), Photophrin II (1 mg) and DOS (100 mg) are weighed out in a small scintillation vial. The round bottom flask is capped and transferred to the sonicator. Decyl methacrylate, hexandioldimethacrylate, and DOS are transferred to the round bottom flask using two 1ml aliquots of ethanol. The solution sonicates for 5-10 minutes and then is transferred back to the stir plate where the slow flow of nitrogen is reinstated and a condenser is added to the flask. The flask is heated to 80°C and allowed to react over night. The solution is suction filtered to obtain the fiberless radiative effectors. After a THF soak and additional filtration the desired dye and ionophores can be added using fresh DOS plasticizer.

Crosslinking of IL13: To produce the fiberless radiative effectors, the dextrate-linked nanospheres are stirred with NHS-biotin and triethylamine in dichloromethane and acetonitrile at room temperature under nitrogen overnight. The fiberless radiative effectors are then isolated by vacuum filtration and dried from toluene azeotrope. The biotinylated fiberless radiative effectors are then incubated with avidin-linked IL13, and incubated for three hours at room temperature. The final product is rinsed and filtered with phosphate buffer.

### Example 3

### Synthesis of Biotinylated Fiberless Radiative Effector

This example describes the synthesis of a biotinylated fiberless radiative effector which can be bound to biotinylated molecular recognition elements via avidin or directly to molecular recognition elements that are directly bound or conjugated to avidin.

Preparation of Acrylamide Nanospheres: The polymerization solution was made by combining 50 mg ruthenium dye, 0.88 g acrylamide, 0.30 g N,N-methylenebis(acrylamide) and 250 µL N-(3-Aminopropyl) methacrylamide hydrochloride (APMA), in 15 mL of 10 mM phosphate buffer, pH 7.4. One mL of polymerization solution was then added to a solution containing 20 mL hexane, 1.8 mmol dioctyl sulfosuccinate sodium salt and 4.24 mmol Brij 30 (4 Lauryl ether), and the two solutions were emulsified by stirring. The polymerization was initiated with 50 µL of a 10% sodium bisulfite solution OR 24 µL of a 10% ammonium persulfate solution and 12 µL N,N,N,N-tetramethylethylenediamine (TEMED), and the solution was stirred at room temperature for 2 hours. Hexane was removed by rotary evaporation and then the effectors were precipitated by the addition of ethanol. Excess surfactant and dye were removed by rinsing with ethanol, to yield a product consisting of 20 and 200 nm probes.

Biotinylation of Acrylamide Nanospheres: Dry nanospheres (27 mg) are suspended in 1000 µL and 3 mg of NHS-biotin is added. The solution is stirred at room temperature for 1 hour, then filtered and rinsed with sodium bicarbonate.

### Example 4

### Synthesis of Fiberless Radiative Effector Biotinylated on the PEG Cloak

This example described the synthesis of fiberless radiative effectors which are biotinylated on their PEG cloaks. Biotinylated molecular recognition elements can be attached to the to the fiberless radiative effector via an avidin linker, or molecular recognition elements bound or conjugated directly avidin can be attached to the fiberless radiative effector.

Preparation of Acrylamide Nanospheres: The polymerization solution was made by combining 50 mg ruthenium dye, 27% acrylamide, 3% N,N-methylenebis(acrylamide), in 1 mL of 10 mM phosphate buffer, pH 7.4. The polymerization solution was then added to a solution containing 20 mL hexane, 1.8 mmol dioctyl sulfosuccinate sodium salt and 4.24 mmol Brij 30 (4 Lauryl ether), and the two solutions were emulsified by stirring. The polymerization was initiated with 50 µL of a 10% sodium bisulfite solution OR 24 µL of a 10% ammonium persulfate solution and 12 µL N,N,N,N-tetramethylethylenediamine (TEMED), and the solution was stirred at room temperature for 2 hours. Hexane was removed by rotary evaporation and then the effectors were precipitated by the addition of ethanol. Excess surfactant and dye were removed by rinsing with ethanol, to yield a product consisting of 20 and 200 nm probes.

PEG Cloaking: Nanospheres are diluted in deionized water to 0.3 % (w/v), then added to an equal volume of -hydroxy PEG (2 % w/v) in water and incubated overnight at room temperature.

Biotin Linkage: The PEG cloaked nanospheres are stirred with NHS-biotin and triethylamine in dichloromethane and acetonitrile at room temperature under nitrogen overnight. The fiberless radiative effectors are then isolated by vacuum filtration and dried from toluene azeotrope.

### Example 5

### Synthesis of Fiberless Radiative Effector Containing a Co-Polymer with Primary Amine Groups

This example describes the synthesis of a fiberless radiative effector containing a co-polymer with primary amine groups. The primary amine groups serve as convenient points of attachment for a variety of cross-linking agents.

Preparation of Acrylamide Nanospheres: The polymerization solution was made by combining 50 mg ruthenium dye, 0.88 g acrylamide, 0.30 g N,N-methylenebis(acrylamide) and 250 µL N-(3-Aminopropyl) methacrylamide hydrochloride (APMA), in 15 mL of 10 mM phosphate buffer, pH 7.4. One mL of polymerization solution was then added to a solution containing 20 mL hexane, 1.8 mmol dictyl sulfosuccinate sodium salt and 4.24 mmol Brij 30 (4 Lauryl ether), and the two solutions were emulsified by stirring. The polymerization was initiated with 50 µL of a 10% sodium bisulfite solution or 24 µL of a 10% ammonium persulfate solution and 12 µL N,N,N,N-tetramethylethylenediamine (TEMED), and the solution was stirred at room temperature for 2 hours. Hexane was removed by rotary evaporation and then the effectors were precipitated by the addition of ethanol. Excess surfactant and dye were removed by rinsing with ethanol, to yield a product consisting of 20 and 200 nm probes.

Linkage of IL13 (or alternate peptide) to Acrylamide Nanospheres: Dry nanospheres (27 mg) are suspended in 1000 µL and 5 mg of IL13 is added. The solution is stirred at room temperature for 1 hour, then filtered and rinsed with sodium bicarbonate. Note that a cross-linking agent is not required because of the intrinsic reactivity of the APMA. However, a homobifunctional or heterobifunctional cross-linking agent could be used if desired.

### Example 6

### Synthesis of Sol-Gel Fiberless Radiative Effector Attached to a Antigen Binding Protein

This example describes the synthesis of a PEG-cloaked sol-gel fiberless radiative effector with an antigen binding protein molecular recognition element (i.e., TAG72).

Preparation of Sol-Gel Nanospheres: A microemulsion solution consisting of 20 mL cyclohexane, 120 µL NH₃H₂O (29% wt.), 100 µL Tetraethylorthosilicate (TEOS), 2.3g polyoxyethylene (4) nonphenyl ether (NP-4) and 50 µg Ruthenium dye was stirred overnight at room temperature. The cyclohexane was removed by rotary evaporation and the fiberless radiative effectors were precipitated by the addition of ethanol and collected by filtration.

PEG Cloaking: The nanospheres were silanized by suspending them in a solution containing 2% aqueous 3-(Trimethoxysilyl)propylmethacrylate adjusted to pH 3.45 and stirring for 2 hours at room temperature. The silanized fiberless radiative effectors were then dialyzed with phosphate buffer and diluted in deionized water to 0.3 % (w/v), then added to an equal volume of -hydroxy PEG (2 % w/v) in water and incubated overnight at room temperature.

TAG72 Antibody Crosslinking: The PEG linked sol-gel nanospheres are stirred with NHS-biotin and triethylamine in dichloromethane and acetonitrile at room temperature under nitrogen overnight. The fiberless radiative effectors are then isolated by vacuum filtration and dried from toluene azeotrope. The biotinylated fiberless radiative effectors are then incubated with avidin linked TAG72, and incubated for three hours at room temperature. The final product is rinsed and filtered with phosphate buffer.

### Example 7

### Synthesis of PEG-Cloaked PVA Fiberless Radiative Effectors

This example describes the synthesis of fiberless radiative effectors from poly(vinyl alcohol).

Preparation of PEG cloaked PVA Nanospheres: PVA (500 mg) was dissolved in 3 mL phosphate buffer (20 mM, pH 7.4) by heating at 90°C. When the PVA solution had cooled to room temperature it was added to 2 mL phosphate buffer which contained 50 mg ruthenium dye and methoxypoly(ethylene glycol) acrylate (2% w/v) and stirred vigorously. This aqueous solution was added to 100 ml silicone oil and the mixture was homogenized for 5 min to form a water-in-oil emulsion. The w/o emulsion was then frozen at -20°C for 20 hours and then allowed to thaw for 4 hours. Three such freezing-thawing cycles were performed to produce a suspension of PVA-PEG nanoparticles in silicone oil. The PVA-PEG nanoparticles were extracted from the silicone oil using acetone and collected by vacuum filtration. The nanoparticles were washed with acetone to remove any residual silicone oil and stored at 4°C.

Cross-linkage to a Peptide: Dry PVA-PEG Fiberless Radiative Effectors (27 mg) are suspended in 1000 µL and 5 mg of IL13 (peptide) is added. The solution is stirred at room temperature for 1 hour, then filtered and rinsed with sodium bicarbonate.

### Example 8

### Synthesis of Radiodynamic Fiberless Radiative Effectors

This example describes the synthesis of radiodynamic fiberless radiative effectors. Polystyrene nanoparticles containing photodynamic compound (*e.g*., Ruthenium dye) can be purchased commercially (Bangs Laboratories, Fisher, IN).

PEG Cloaking: The polystyrene nanospheres were reacted with the macromonomer methoxypoly(ethylene glycol) acrylate in the molar ratio 1:0.025 using potassium persulfate as the initiator. The polystyrene-PEG (PS-PEG) particles were then filtered and dialyzed.

Radiodynamic Labelling: The PS-PEG particles were surface labelled with NaI-125 (Amersham Int'l. U.K., an iodine radiolabel). NaI-125 (2.5 µL, 3.7 GBq/ml) was mixed with 1 mL of PS-PEG particle dispersion (2% w/v) and exposed to a Cesium-137 source for 48 hours. After irradiation, excess free iodine was removed by dialysis.

Crosslinking of Peptide and Avidin: The radiolabelled PS-PEG nanospheres are stirred with NHS-biotin and triethylamine in dichloromethane and acetonitrile at room temperature under nitrogen overnight. The fiberless radiative effectors are then isolated by vacuum filtration and dried from toluene azeotrope. The biotinylated fiberless radiative effectors are then incubated with avidin linked IL13 (or some other peptide), and incubated for three hours at room temperature. The final product is rinsed and filtered with phosphate buffer.

### Example 9

### Synthesis of Fiberless Radiative Effector Containing a Gamma Ray Producer

This example describes the synthesis of a fiberless radiative effector containing a gamma ray producer. This fiberless radiative effector may be used in conjunction with the radiodynamic fiberless radiative effector described in Example 8 or by itself.

Preparation of Acrylamide Nanospheres: Fiberless radiative effectors are produced using the methods described in Example 1, except that Ruthenium dye in the polymerization solution is replaced with a gamma ray producer (¹¹¹In-oxine, 37 MBq/ml, Amersham Int'l) and ascorbic acid (35 mg) as an antioxidant.

PEG Cloaking: Nanospheres are diluted in deionized water to 0.3 % (w/v), then added to an equal volume of -hydroxy PEG (2 % w/v) in water and incubated overnight at room temperature.

Biotin linkage: The PEG cloaked nanospheres are stirred with NHS-biotin and triethylamine in dichloromethane and acetonitrile at room temperature under nitrogen overnight. The fiberless radiative effectors are then isolated by vacuum filtration and dried from toluene azeotrope.

### Example 10

### Synthesis of Fiberless Radiative Effector Containing Magnetic Material

This example describes the synthesis of fiberless radiative effectors containing magnetic material. These fiberless radiative effectors may be imaged by magnetic resonance and can be used to both destroy a biological target and monitor the effect of the therapy. Briefly, fiberless radiative effectors are prepared as in Examples 1 or 2, but with the addition of monocrystalline iron oxide nanoparticles (MION) at the polymerization solution stage.

### Example 11

### Synthesis of Fiberless Radiative Effectors Contained in Liposome Cloak

This example describes the synthesis of fiberless radiative effectors comprising a cloak. The liposome material includes a PEG derivative which can be biotinylated so that molecular recognition elements can be attached to the liposome for biological targeting.

PEG-linked Liposomes Containing Fiberless Radiative Effectors Preparation: A mixture of phosphatidylcholine (PC) and cholesterol (1:1 molar ratio) was mixed with 7.4 mol% of PEG-PE (PEG linked dioleoylphosphatidylethanolamine) in dichloromethane/ethanol in a round bottom flask. The organic solvent was evaporated under nitrogen, then vacuum desiccated for 2 hrs. Normal saline containing a suspension of nanospheres (5 %) was added and the lipid suspension was vortexed for 2 min and extruded ten times through two stacked Nucleopore polycarbonate filters (400 nm pore size).

Crosslinking of IL13, peptide or antibody: Fiberless radiative effectors are produced by stirring the PEG linked liposomes with NHS-biotin in saline at room temperature under nitrogen overnight. The biotinylated liposomes are then incubated with avidin linked IL13, and incubated for three hours at room temperature. The final product is rinsed and filtered with phosphate buffer.

### Example 12

### Synthesis of Fiberless Radiative Effector Containing a Bioluminescent Protein

This example describes the synthesis of fiberless radiative effectors that contain a bioluminescent protein. These fiberless radiative effectors are used for imaging or to provide a light source for excitation of fiberless radiative effectors containing a photodynamic compound. Briefly, these fiberless radiative effectors are fabricated using the procedure outlined in Example 6 above to prepare PEG cloaked sol-gel fiberless radiative effectors, except that 25 µg luciferase for 50 µg Ruthenium dye. Conjugation of the PEG cloaked fiberless radiative effectors to biotin is completed by following the procedure described in Example 4.

### Example 13

### Photodynamic Fiberless Radiative Effector Mediated Cell Death

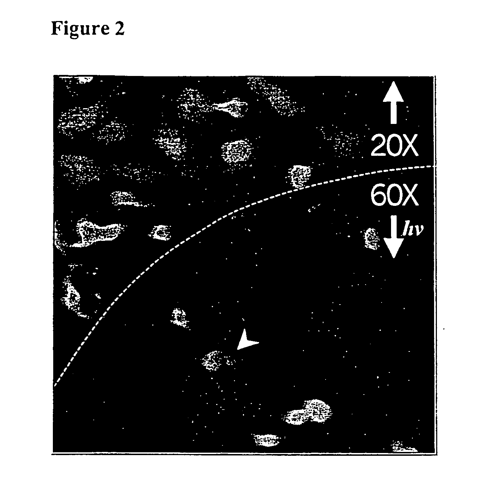

This example demonstrates that fiberless radiative effectors containing a photodynamic compound are toxic to cells. Human SY5Y neuroblastoma cells lipofected with fiberless radiative effectors containing oxygen-sensitive ruthenium red dye were evaluated for indices of cell death by confocal microscopy upon exposure to laser light. Cells were exposed for 30 min to laser light using the 488 nm line of an Ar-Kr laser on a laser scanning confocal microscope by focusing the beam (attenuated to 60% power and further attenuated by 20% using barrier filters) through a 60X 1.4/NA objective. Subsequent to laser exposure cells were loaded for 15 min at 37°C, with the viability indicator dye calcein (AM) and the cell death indicator dye propidium iodide, and imaged by confocal microscopy using a 20X 0.75/NA objective (488EX/525EM for calcein, 568EX/590EM). The dotted line indicates (See Fig. 2) the demarcation between cells exposed to the light path (60 or 40X) or not exposed (20X). Arrowheads indicate dead (propidium iodide) or dying (morphologic appearance of apoptosis) cells. Cells were grown to confluence prior to illumination. The density of cells within the area of illumination was severely reduced. Even though several of the cells in the irradiated area are not yet dead, they displayed many signs of phototoxicity including "balling-up" of cells and retraction of any neurites and phylopodia which may have developed during culture. Analogous results have been obtained with C6 glioma cells using ruthenium nanospheres. Despite the fact that the neuroblastoma and glioma cells in culture have very different susceptibilities (SY5Y cells do not express Bel-XL in response to oxidative stress), they respond similarly to the photodynamic effects of fiberless radiative effectors.

### Example 14

### Non-Specific Liposomal Delivery of PEBBLEs

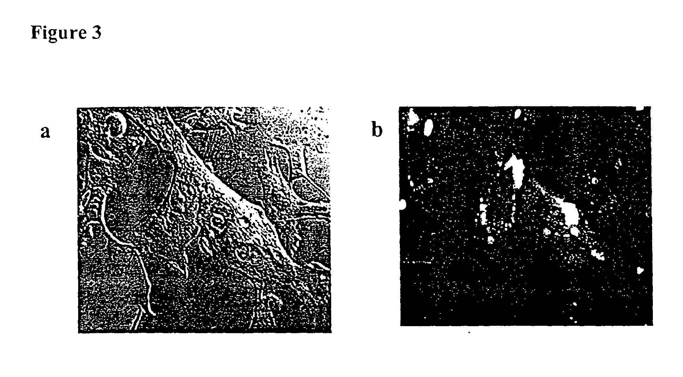

Liposomal delivery is accomplished using commercially available liposomes called ESCORT (composed of a 1:1 mixture of cationic lipids, N-[1-(2,3-dioleoyloxy)propyl]-N,N,N-trimethylammonium chloride and dioleoyl phosphatidylethanolamine). The liposomes incorporate into the cell membrane and empty their contents (*e.g.,* PEBBLEs; Probes Encapsulated By Biologically Localized Embedding) into the cytoplasm of the cell. Liposomal delivery has proven to be an effective method for inserting a large amount of PEBBLEs into cells, unlike the gene gun which only injects one or two PEBBLEs into a cell. Additionally, a large number of cells can be injected at once, in contrast to pico-injection, which is useful only on a cell-by-cell basis. ESCORT was used effectively to insert calcium selective PEBBLEs into neuroblastoma cells (Fig. 3; (a) Nomarski illumination; (b) fluorescence image with excitation at 590 nm).

### Example 15

### In Situ Imaging of Tumors by MRI

In this example, MRI was used to image non-specific monocrystalline iron oxide (MION) particles in tumor cells in rats. In these experiments, rats with intracerebral 9L tumors were injected in the tail vein with MION particles at a dose of 15 mg Fe/kg body weight. T1 and T2-weighted MR imaging was performed over time pre-and 1, 2 and 3 days post-MION administration on a 7-Tesla MR system. A time series of *in vivo* T1-weighted MR images from a single animal harboring a 9L tumor were collected. At 24 hours following MION administration, the contrast in the tumor but not in the contralateral brain tissue was dramatically altered by the presence of MION as evidenced by the shortening of the T1 relaxation time resulting in an increase in the MR T1 signal intensity. At 2 days following MION administration, the change in signal contrast had almost completely returned to that observed in the pre-MION tumor except for the nanoparticles left in the necrotic region of the tumor (right side). Next, MR images were from the same animal, but with T2-weighting. In this case (T2-weighted imaging), the presence of MION was revealed by the loss of signal intensity and the fact that the tumor turns dark to black in regions where the MION particles have accumulated. T2-weighted images appear a bit more sensitive as the change in signal persists into days 2-3 post MION-administration. Histological examination of sections stained for hematoxylin and DAB amplified Perls' confirmed the dynamic time-course for uptake and elimination of MION in the tumor tissue and the lack of entrance into the normal brain parenchyma. The selectivity of entrance into the tumor tissue is due to the disrupted blood brain barrier within the tumor mass. This data reveals that MR imaging, especially T2-weighted imaging can clearly be used to non-invasively follow the uptake and clearance of nanoparticles within tumor tissue. It is contemplated that targeting prolongs the half-life of nanoparticles in the tumor, thereby enhancing imaging in therapeutic regimens.

### Example 16

### In Situ Evaluation of Tumor Therapy by MRI

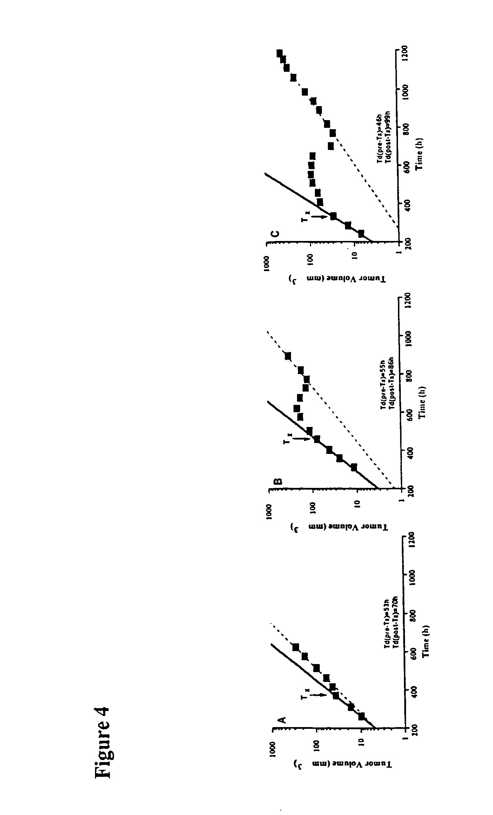

To demonstrate the utility of MRI for non-invasive quantitation of therapeutic efficacy, rats with intracerebral 9L brain tumors were treated with BCNU and used serial MRI to follow tumor growth over time. In summary, these experiments revealed that tumor cell log kills can be quantitated in individual animals. Shown in Figures 4 A, B and C are plots of representative intracranial tumor volumes versus time, each plot taken from a single animal, both pre- and post-treatment for 0.5, 1, and 2 LD10 doses of BCNU. The individual volume measurements are shown along with the pre- (solid line) and post-treatment (dashed line) exponential regrowth curve fits following treatment with (A) 0.5, (B) 1.0, and (C) 2.0 times LD10 BCNU. Pre- and post-BCNU doubling times (td) are also shown, revealing a substantial decrease in growth rate during tumor regrowth.

Tumor growth slowed immediately following BCNU treatment and the time to regrowth increased with increasing BCNU dose. An exceptional finding was that tumors re-grow at a significantly slower rate following treatment. This fact was previously unknown, even after extensive research reports in the literature using the 9L tumor model to study the effects of BCNU. Log kill values were obtained from these data for each individual animal by extrapolation of the regrowth line back to the time of treatment as denoted by Tx in Figure 4. Using MRI tumor volumetric data, it was found that 9L tumor log kill values were 0.2±0.1 (±S.D., n=5), 1.0±0.3 (n=4) and 1.7±0.6 (n=6), respectively for 0.5, 1 and 2 LD10 BCNU doses which are considerably less (Ross *et al.,* PNAS 95:7012-17 [1998]) than the 1.7, 3.2 and 3.7 log kills derived from traditional measurements (Rosenblum *et al.,* J. Neurosurg. 46:145-54 [1997]). It is not intended that the present invention be limited to particular mechanism of action. Indeed, an understanding of the mechanism is not necessary to make and use the present invention. However, the differences in calculated cell kills are largely attributable to the fact that it was assumed that regrowth and untreated tumor growth kinetics were equivalent. Moreover, the slower tumor re-growth kinetics following treatment reveals that increases in animal survival cannot be attributed solely to the proportion of tumor cell killed. While it is true that more cells are killed at higher doses of BCNU, this does not appear to be the dominant reason for the delay in tumor progression. It appears that the slower growth rate following treatment effects tremendously the time to repopulate the tumor. This data shows the power of the MRI approach for following the effects of therapy on intracranial tumor growth.

### Example 17

### Detection of Biological Targets with Fiberless Radiative Effector Containing Luciferase

This example describes the utility of fiberless radiative effectors containing the useful radiative beacon, luciferase. Confocal fluorescence images of DNPs containing luciferase encapsulated in sol gel matrices are obtained using the Olympus AX70 fluorescence microscope fitted with a Spectramaster monochromater and spinning disc Confocal attachment. Green or red fluorescence of luciferin are excited at the appropriate wavelength using the liquid fiber-optic light guide of the monochromator. Emitted fluorescence are collected using a 60X-1.4 NA oil-immersion objective lens. Data is acquired and analyzed using the LSR Merlin and Esprit Software on a dedicated Dell 400 MHz Pentium II MMX computer with 1Gb RAM. Sol-gel fiberless radiative effectors are delivered to cells in culture using liposomal techniques. The specificity of light produced is determined by depleting cells of ATP using p-nitrophenol to uncouple mitochondria.

### Example 18

### Targeted Destruction of Tumor Cells

This example describes the destruction of tumor cells by fiberless radiative effectors conjugated to tumor cell specific molecular recognition elements. The RGD peptide (CDCRGDCFC; SEQ ID NO:1) and IL13 will be conjugated to fiberless radiative effectors as described.

*Confocal Microscopy Analysis of Apoptosis:* To evaluate onset of apoptosis, cultured C6 glioma and SY5Y neuroblastoma cells grown on 22 mm glass coverslips are incubated and exposed to DNPs and loaded for 15 min with 1 M calcein acetoxymethyl ester (AM). Following dye-loading, cells are washed once with incubation buffer (Dulbecco's phosphate buffered saline containing CaCl₂, 0.1 g/L, glucose, 1 g/L and sodium pyruvate, 0.036 g/L), mounted on the microscope stage in incubation buffer containing 3 M propidium iodide and incubated at 37°C. Sequential confocal images will be obtained for cytosolic (calcein) and nuclear (propidium iodide) staining to evaluate morphologic changes associated with apoptosis. For longer treatment time-points (beyond 1 hr), cells are loaded with dyes as described above and single confocal images obtained for calcein and propidium iodide. Confocal fluorescence images of calcein and propidium iodide are obtained using the Olympus AX70 fluorescence microscope fitted with a Spectramaster monochromater and Confocal attachment. Green fluorescence of calcein and red fluorescence of propidium iodide is excited simultaneously at 488 and 568-nm using the liquid fiber-optic light guide of the monochromator. Emitted fluorescence is monitored using a 60X-1.4 NA oil-immersion objective lens. Data will be acquired and analyzed using the LSR Merlin and Esprit Software on a dedicated Dell 400 MHz Pentium II MMX computer with 1Gb RAM.

*DNA Laddering Analysis of Apoptosis:* Cells treated as described above are harvested for genomic DNA isolation using the QiaPrep DNA isolation system (Qiagen, Chatsworth, CA). Genomic DNA samples are resolved by electrophoresis on 1.5% agarose gels in Tris-Borate-EDTA (1X TBE) buffer containing ethidium bromide (50 ng/ml) for 1.5 hours at 50 mV. The appearance of discreet 160 kb bands in DNA samples from cells undergoing apoptosis is visualized by UV illumination and polaroid photography.

### Example 19

### In Situ Monitoring of Fiberless Radiative Effector Therapy

This example describes the *in situ* monitoring in rats of tumor therapy using fiberless radiative effectors.

*Cell Culture Conditions:* Both SCC VII (mouse squamous cell carcinoma of the head and neck) and 9L (human gliosarcoma) tumor cells are grown as monolayers in 75-cm² sterile plastic flasks in modified Eagle's minimum essential medium with 10% fetal calf serum. Cells are cultured in an incubator at 37°C in an atmosphere containing 95% air and 5% CO₂ until confluent. The cells are harvested, counted and concentrated in serum-free media for tumor implantation.

*Stable Transfection:* Stable 9L and SCCVII cell lines are constructed which stably carry and express the luciferase gene. Animals are implanted with these lines at their respective sites (brain and submental region of the mouth) and imaged twice a week for six weeks. To validate that the bioluminescence results are reproducible measures of tumor burden, MRI data is collected.

Both cell lines are transfected with pgLUC/CMV by the calcium phosphate precipitation method. Exponentially growing cells will be split 1:10 onto 10-cm tissue culture dishes the day before transfection with 30 µg of plasmid. Stable transfectants are selected by growth in 500 µM G418 for approximately two weeks. Luciferase expression from polyclonal transfectants from multiple transfections is analyzed as described below. Luciferase expression from monoclonal lines isolated from serial dilution is assayed in a 96-well plate using a Perkin Elmer LS50B bioluminometer.

*Induction of Intracranial and Head & Neck Tumors:* Intracerebral brain tumors are induced in anesthetized male Fischer-344 rats. A small skin incision is made through the scalp and a 1 mm-diameter hole drilled through the cranium using a high speed drill. A suspension of 1x10⁵ tumor cells in 10 µl serum-free culture media is slowly injected into the right frontal lobe at a depth of 2.5 mm from the dural surface. The burr hole is filled with bone wax and the incision closed with suture.

In the experiments described here, C3H mice are utilized as hosts and SCCVII cells as the model squamous cell tumor. Cells are harvested from 150 mm tissue culture dishes by scraping, washing in PBS, and resuspending in media at 1.2 x 10⁸ cells/ml. C3H mice aged 6-10 weeks and weighing 25-30 grams are anesthetized by i.p. injection of 0.5 ml avertin at a concentration of 20 mg/ml. Fifty µl of the cell suspension will be injected into the submental region using a 26 gauge syringe.

*Magnetic resonance imaging (MRI):* All *in vivo* MR experiments are performed on a Varian Imaging system equipped with a 7.0 Tesla, 18.3 cm horizontal bore magnet (300 MHz proton frequency). Rat brains harboring intracerebral tumors are imaged at approximately 10-30 days following cell implantation in two day intervals. Each MRI session includes: (a) Single slice gradient-recalled-echo scanning with 1 mm "saturation cross-hairs" imprinted on the axial and coronal images to facilitate rapid and reproducible positioning of the coil and rigid animal support rig (ear posts and bite bar). T2-weighted images through the rat brain are produced using the following parameters: TR/TE = 3500/60, FOV= 30 x 30 mm using a 128 x 128 matrix, slice thickness = 0.5 mm, number of slices and slice separation = 0.8 mm.

*Bioluminescent Imaging of Tumors:* A Hamamatsu intensified CCD camera (Model C2400-32) is used for imaging of tumor bearing animals. Tumor cells constitutively expressing luciferase are grown in the brain or neck of animals. At the time of imaging, animals are injected with luciferin i.p. at a dose of 150 mg/kg as described previously (Contag *et al., supra*)*.* Animals are anesthetized and placed in the imaging chamber and data will be acquired for 5-10 minutes depending upon tumor burden. Data is processed off-line using software provided by Dr. Chris Contag, Stanford University.

As is evident from the above, the present invention provides a viable alternative strategy and reagents for photodynamic therapy. The fiberless radiative effectors of the present invention can be administered in low doses, so that collateral damage of adjacent tissues or cells is low, and so that cutaneous photosensitivity should be reduced. Various modifications and variations of the described method and system of the invention will be apparent to those skilled in the art.

## Claims

1. A composition comprising a molecular recognition element attached to a fiberless radiative effector comprising a polymer, **characterized in that** said molecular recognition element is selected from the group consisting of a peptide, protein, carbohydrate, nucleic acid and biotin capable of binding to a target, said target being selected from the group consisting of a cell surface protein, a cell surface receptor, an extracellular matrix protein, a viral coat protein, and a bacterial cell wall protein, said polymer incorporating an agent, wherein said agent produces a toxic diffusable substance upon stimulation from an energy source.

2. The composition of Claim 1, wherein said molecular recognition element is a peptide containing a motif selected from the group consisting of RGD, NGR and GSL.

3. The composition of Claim 1, wherein said polymer is selected from poly(vinyl chloride), poly(vinyl chloride) carboxylated and poly(vinyl chloride-co-vinyl acetate co-vinyl) alcohols.

4. The composition of Claim 1, wherein said fiberless radiative effector further comprises a plasticizer.

5. The composition of Claim 1, wherein said toxic substance is selected from the group consisting of singlet oxygen, hydroxyl radicals, superoxide radicals and nitric oxide.

6. The composition of Claim 1, further comprising a cloaking material.

7. The composition of Claim 6, wherein said cloaking material is selected from poly(ethylene glycol) and a liposome.

8. The composition of Claim 6, wherein said cloaking material is a liposome, said liposome containing a plurality of said fiberless radiative effectors.

9. The composition of Claim 7, wherein said molecular recognition element is attached to said liposome.

10. Use of a composition according to any of claims 1 to 9 for the manufacture of a medicament for the treatment of tumors displaying a biological target.

11. The use of Claim 10, wherein said tumor comprises malignant glioma cells and said molecular recognition element comprises IL13.

12. The use of Claim 10, wherein said agent comprises a photosensitizer.

13. The use of Claim 12, where said photosensitizer dye is selected from Photofrin, Ru-diphenylphenanthroline, Tris(1-10-phenanthroline)ruthenium(II) chloride), tin ethyl etiopurpurin, protoporphyrin IX, chloroaluminum phthalocyanine, tetra(M-hydroxyphenyl)chlorin.

14. The use of Claim 13, wherein said photosensitizer dye is capable of producing singlet oxygen when exposed to a light source.

15. The use of Claim 10, wherein said composition further comprises a second fiberless radiative effector comprising a second molecular recognition element, said second effector being capable of forming a second effector-target complex.

16. The use of Claim 15, wherein said agent comprises a photosensitizer.

17. The use of Claim 16, wherein said second fiberless radiative effector comprises a radioactive element.

18. Use of a composition according to any of the claims 1 to 9 and a marker for the manufacture of a medicament for the diagnosis of tumors displaying a biological target.

19. The use of Claim 18, wherein said marker is a protein that catalyzes a luminescent reaction.

20. The use of Claim 18, wherein said first fiberless radiative effector-target complex is capable of producing luminescence when exposed to a substrate.

21. The use of Claim 18, wherein said marker is a magnetic material.

22. The use of Claim 21, wherein said magnetic material can be detected by magnetic resonance imaging.

23. A composition comprising a molecular recognition element attached to a liposome, **characterized in that** said molecular recognition element is selected from the group consisting of a peptide, protein, carbohydrate, nucleic acid and biotin capable of binding to a target, said target selected from the group consisting of a cell surface protein, a cell surface receptor, an extracellular matrix protein, a viral coat protein, and a bacterial cell wall protein, said liposome containing a plurality of fiberless radiative effectors comprising a polymer, said polymer incorporating an agent, wherein said agent produces a toxic diffusable substance upon stimulation from an energy source.

24. The composition of Claim 23, wherein said molecular recognition element comprises a peptide containing a motif selected from the group consisting of RGD, NGR and GSL.

25. The composition of Claim 23, wherein said liposome further comprises poly(ethylene glycol).

26. The composition of Claim 23, wherein said polymer is selected from poly(vinyl chloride), poly(vinyl chloride) carboxylated and poly(vinyl chloride-co-vinyl acetate co-vinyl) alcohols.

27. The composition of Claim 23, wherein said fiberless radiative effector further comprises a plasticizer.

28. The composition of Claim 23, wherein said toxic substance is selected from the group consisting of singlet oxygen, hydroxyl radicals, superoxide radicals and nitric oxide.

## Patentansprüche

1. Zusammensetzung, ein Element zur molekularen Erkennung umfassend, welches mit einem Strahlungseffektor ohne Fiber verbunden ist, der ein Polymer umfasst, **dadurch gekennzeichnet, dass** das Element zur molekularen Erkennung aus der Gruppe gewählt wird, die aus einem Peptid, Protein, Kohlenhydrat, Nukleinsäure und Biotin besteht, welche dazu fähig sind, sich an eine Zielregion zu binden, wobei die Zielregion aus der Gruppe gewählt wird, die aus einem Zelloberflächenprotein, einem Zelloberflächenrezeptor, einem extrazellulären Matrixprotein, einem viralen Hüllprotein und bakteriellen Zellwandprotein besteht, und das Polymer ein Mittel enthält, wobei dieses Mittel nach Anregung durch eine Energiequelle eine diffusionsfähige giftige Substanz produziert.

2. Zusammensetzung nach Anspruch 1, wobei es sich bei dem Element zur molekularen Erkennung um ein Peptid handelt, welches ein Strukturprinzip enthält, das aus der Gruppe gewählt wird, die aus RGD, NGR und GSL besteht.

3. Zusammensetzung nach Anspruch 1, wobei das Polymer aus Poly(vinylchlorid), carboxylierten Poly(vinylchlorid)-Alkoholen sowie Poly(vinylchloridcovinylacetat covinyl)-Alkoholen gewählt wird.

4. Zusammensetzung nach Anspruch 1, wobei der Strahlungseffektor ohne Fiber darüber hinaus einen Weichmacher umfasst.

5. Zusammensetzung nach Anspruch 1, wobei die giftige Substanz aus der Gruppe gewählt wird, die aus Singulett-Sauerstoff, Hydroxylradikalen, Superoxidradikalen und Stickstoffoxid besteht.

6. Zusammensetzung nach Anspruch 1, die darüber hinaus ein Hüllmaterial umfasst.

7. Zusammensetzung nach Anspruch 6, wobei das Hüllmaterial aus Poly(ethylenglykol) und einem Liposom gewählt wird.

8. Zusammensetzung nach Anspruch 6, wobei es sich bei dem Hüllmaterial um ein Liposom handelt und dieses Liposom mehrere Strahlungseffektoren ohne Fiber enthält.

9. Zusammensetzung nach Anspruch 7, wobei das Element zur molekularen Erkennung mit dem Liposom verbunden ist.

10. Verwendung einer Zusammensetzung nach einem der Ansprüche 1 bis 9 zur Herstellung eines Medikaments, das zur Behandlung von Tumoren dient, die eine biologische Zielregion aufweisen.

11. Verwendung nach Anspruch 10, wobei der Tumor maligne Gliomazellen umfasst und das Element zur molekularen Erkennung IL13 umfasst.

12. Verwendung nach Anspruch 10, wobei das Mittel einen Photosensibilisator umfasst.

13. Verwendung nach Anspruch 12, wobei Photosensibilisator-Farbstoff aus Photofrin, Ru-Diphenylphenanthrolin, Tris(1,10-phenanthrolin)ruthenium(II)chlorid, Zinnethyletiopurpurin, Protoporphyrin IX, Chloraluminiumphthalocyanin und Tetra(M-hydroxyphenyl)chlorin gewählt wird.

14. Verwendung nach Anspruch 13, wobei der Photosensibilisator-Farbstoff dazu fähig ist, Singulett-Sauerstoff zu produzieren, wenn er einer Lichtquelle ausgesetzt wird.

15. Verwendung nach Anspruch 10, wobei die Zusammensetzung weiter einen zweiten Strahlungseffektor ohne Fiber umfasst, der ein zweites Element zur molekularen Erkennung umfasst, wobei der zweite Effektor dazu fähig ist, einen zweiten Effektor-Ziel-Komplex zu bilden.

16. Verwendung nach Anspruch 15, wobei das Mittel einen Photosensibilisator umfasst.

17. Verwendung nach Anspruch 16, wobei der zweite Strahlungseffektor ohne Fiber ein radioaktives Element umfasst.

18. Verwendung einer Zusammensetzung nach einem der Ansprüche 1 bis 9 sowie eines Markers, um ein Medikament zur Diagnose von Tumoren, die eine biologische Zielregion aufweisen, herzustellen.

19. Verwendung nach Anspruch 18, wobei es sich bei dem Marker um ein Protein handelt, das eine Lumineszenzreaktion katalysiert.

20. Verwendung nach Anspruch 18, wobei der erste Komplex aus Zielregion und Strahlungseffektor ohne Fiber dazu fähig ist, Lumineszenz zu erzeugen, wenn er einem Substrat ausgesetzt ist.

21. Verwendung nach Anspruch 18, wobei es sich bei dem Marker um magnetisches Material handelt.

22. Verwendung nach Anspruch 21, wobei das magnetische Material mit Magnetresonanz-Bildgebungsverfahren detektiert werden kann.

23. Zusammensetzung, die ein Element zur molekularen Erkennung umfasst, das mit einem Liposom verknüpft ist, **dadurch gekennzeichnet, dass** das Element zur molekularen Erkennung aus der Gruppe gewählt wird, die aus einem Peptid, Protein, Kohlenhydrat, Nukleinsäure und Biotin besteht, welche dazu fähig sind, sich an eine Zielregion zu binden, wobei die Zielregion aus der Gruppe gewählt wird, die aus einem Zelloberflächenprotein, einem Zelloberflächenrezeptor, einem extrazellulären Matrixprotein, einem viralen Hüllprotein und bakteriellen Zellwandprotein besteht, und das Liposom mehrere Strahlungseffektoren ohne Fiber enthält, die ein Polymer umfassen, welches ein Mittel enthält, wobei dieses Mittel nach Anregung durch eine Energiequelle eine diffusionsfähige giftige Substanz produziert.

24. Zusammensetzung nach Anspruch 23, wobei das Element zur molekularen Erkennung ein Peptid umfasst, welches ein Strukturprinzip enthält, das aus der Gruppe gewählt wird, die aus RGD, NGR und GSL besteht

25. Zusammensetzung nach Anspruch 23, wobei das Liposom darüber hinaus Poly(ethylenglykol) umfasst.

26. Zusammensetzung nach Anspruch 23, wobei das Polymer aus Poly(vinylchlorid), carboxylierten Poly(vinylchlorid)-Alkoholen sowie Poly(vinylchlorid-covinylacetat covinyl)-Alkoholen gewählt wird

27. Zusammensetzung nach Anspruch 23, wobei der Strahlungseffektor ohne Fiber darüber hinaus einen Weichmacher umfasst.

28. Zusammensetzung nach Anspruch 23, wobei die giftige Substanz aus der Gruppe gewählt wird, die aus Singulett-Sauerstoff, Hydroxylradikalen, Superoxidradikalen und Stickstoffoxid besteht.

## Revendications

1. Composition comprenant un élément de reconnaissance moléculaire attaché à un effecteur radiatif sans fibres comprenant un polymère, **caractérisé en ce que** ledit élément de reconnaissance moléculaire est sélectionné dans le groupe constitué par un peptide, une protéine, un hydrate de carbone, un acide nucléique et la biotine capable de se lier à une cible, ladite cible étant sélectionnée dans le groupe constitué par une protéine de la surface cellulaire, un récepteur de la surface cellulaire, une protéine de la matrice extracellulaire, une protéine d'enveloppe virale et une protéine de la paroi cellulaire bactérienne, ledit polymère incorporant un agent, dans lequel ledit agent produit une substance toxique diffusible par stimulation en provenance d'une source d'énergie.

2. Composition selon la Revendication 1, dans laquelle ledit élément de reconnaissance moléculaire est un peptide contenant un motif sélectionné dans le groupe constitué par RGD, NGR et GSL.

3. Composition selon la Revendication 1, dans laquelle ledit polymère est sélectionné parmi le poly(chlorure de vinyle), le poly(chlorure de vinyle) carboxylé et les alcools de poly(chlorure de vinyle-co-acétate de vinyle co-vinyle).

4. Composition selon la Revendication 1, dans laquelle ledit effecteur radiatif sans fibres comprend en outre un agent plastifiant.

5. Composition de la Revendication 1, dans laquelle ladite substance toxique est sélectionnée dans le groupe constitué par l'oxygène singulet, les radicaux hydroxyle, les radicaux superoxyde et l'oxyde nitrique.

6. Composition selon la Revendication 1, comprenant en outre un matériau enveloppant.

7. Composition de la Revendication 6, dans lequel ledit matériau enveloppant est sélectionné parmi le poly(éthylène glycol) et un liposome.

8. Composition selon la Revendication 6, dans laquelle ledit matériau enveloppant est un liposome, ledit liposome contenant une pluralité desdits effecteurs radiatifs sans fibre.

9. Composition selon la Revendication 7, dans laquelle ledit élément de reconnaissance moléculaire est attaché audit liposome.

10. Utilisation d'une composition selon l'une quelconque des Revendications 1 à 9 pour la fabrication d'un médicament pour le traitement de tumeurs présentant une cible biologique.

11. Utilisation d'une composition selon la Revendication 10, dans laquelle ladite tumeur comprend des cellules de gliome malin et ledit élément de reconnaissance moléculaire comprend IL13.

12. Utilisation d'une composition selon la Revendication 10, dans laquelle ledit agent comprend un photosensibilisateur.

13. Utilisation d'une composition selon la Revendication 12, dans laquelle ledit colorant photosensibilisateur est sélectionné parmi Photofrine, Ru-diphényl-phénanthroline, chlorure de Tris(1-10-phénanthroline)ruthénium(II), éthyl étiopurpurine d'étain, protoporphyrine IX, chloroaluminium phtalocyanine, tétra(M-hydroxyphényl)chlore.

14. Utilisation d'une composition selon la Revendication 13, dans laquelle ledit colorant photosensibilisateur est capable de produire de l'oxygène singulet lorsqu'il est exposé à une source lumineuse.

15. Utilisation d'une composition selon la Revendication 10, dans laquelle ladite composition comprend en outre un second effecteur radiatif sans fibres comprenant un second élément de reconnaissance moléculaire et étant capable de former un second complexe effecteur-cible.

16. Utilisation d'une composition selon la Revendication 15, dans laquelle ledit agent comprend un photosensibilisateur.

17. Utilisation d'une composition selon la Revendication 16, dans laquelle ledit second effecteur radiatif sans fibres comprend un élément radioactif.

18. Utilisation d'une composition selon l'une quelconque des Revendications 1 à 9 et d'un marqueur pour la fabrication d'un médicament pour le diagnostic de tumeurs présentant une cible biologique.

19. Utilisation d'une composition selon la Revendication 18, dans laquelle ledit marqueur est une protéine qui catalyse une réaction luminescente.

20. Utilisation d'une composition selon la Revendication 18, dans laquelle ledit complexe cible-effecteur radiatif sans fibres est capable de produire de la luminescence lorsqu'il est exposé à un substrat.

21. Utilisation d'une composition selon la Revendication 18, dans laquelle ledit marqueur est un matériau magnétique.

22. Utilisation d'une composition selon la Revendication 21, dans laquelle ledit matériau magnétique peut être détecté par imagerie de résonance magnétique.

23. Composition comprenant un élément de reconnaissance moléculaire attaché à un liposome, **caractérisé en ce que** ledit élément de reconnaissance moléculaire est sélectionné dans le groupe constitué par un peptide, une protéine, un hydrate de carbone, un acide nucléique et la biotine, capable de se lier à une cible, ladite cible sélectionnée dans le groupe constitué par une protéine de la surface cellulaire, un récepteur de la surface cellulaire, une protéine de la matrice extracellulaire, une protéine de l'enveloppe virale, et une protéine de la paroi cellulaire bactérienne, ledit liposome contenant une pluralité d'effecteurs radiatifs sans fibres comprenant un polymère, ledit polymère incorporant un agent, où ledit agent produit une substance diffusible toxique par stimulation provenant d'une source d'énergie.

24. Composition selon la Revendication 23, dans laquelle ledit élément de reconnaissance moléculaire comprend un peptide contenant un motif sélectionné dans le groupe constitué par RGD, NGR et GSL.

25. Composition selon la Revendication 23, dans laquelle ledit liposome comprend en outre du poly(éthylène glycol).

26. Composition selon la Revendication 23, dans laquelle ledit polymère est sélectionné parmi le poly(chlorure de vinyle), le poly(chlorure de vinyle) carboxylé et les poly(chlorure de vinyle)-co-acétate de vinyle co-vinyle) alcools.

27. Composition selon la Revendication 23, dans laquelle ledit effecteur radiatif sans fibres comprend en outre un agent plastifiant.

28. Composition selon la Revendication 23, dans laquelle ladite substance toxique est sélectionnée dans le groupe constitué par l'oxygène singulet, les radicaux hydroxyle, les radicaux superoxyde et l'oxyde nitrique.
